# EUROPEAN PATENT SPECIFICATION

(11) **EP 2 310 505 B1**
(45) Date of publication and mention of the grant of the patent: **09.08.2017**
(21) Application number: 09772365.4
(22) Date of filing: 25.06.2009
(51) Int. Cl.: C12N 15/11

(54) **ANTIDOTE OLIGOMERS**
ANTIDOT-OLIGOMERE
OLIGOMÈRES D'ANTIDOTE

(30) Priority: 30.06.2008 US 77096 P; 22.01.2009 US 146326 P
(43) Date of publication of application: 20.04.2011
(73) Proprietor: Roche Innovation Center Copenhagen A/S, 2970 Hørsholm (DK)
(72) Inventor: HANSEN, Jens, Bo, Rhode, DK-2920 Charlottenlund (DK); NIELSEN, Niels Fisker, 2800 Kgs. Lyngby (DK)
(74) Representative: Hansen, Marianne Rudolph
(86) International application number: PCT/EP2009/057953
(87) International publication number: WO 2010/000665

(56) References cited:
- WO-A-02/096926
- WO-A-2009/061841
- JEPSEN J S ET AL: "LNA-ANTISENSE RIVALS SIRNA FOR GENE SILENCING" CURRENT OPINION IN DRUG DISCOVERY AND DEVELOPMENT, CURRENT DRUGS, LONDON, GB, vol. 7, no. 2, 1 January 2004 (2004-01-01), pages 188-194, XP009083873 ISSN: 1367-6733
- ONEY S ET AL: "Antidote-controlled platelet inhibition targeting von Willebrand factor with aptamers." OLIGONUCLEOTIDES FALL 2007, vol. 17, no. 3, October 2007 (2007-10), pages 265-274, XP002552136 ISSN: 1545-4576
- LEE J F ET AL: "Aptamer therapeutics advance" CURRENT OPINION IN CHEMICAL BIOLOGY, CURRENT BIOLOGY LTD, LONDON, GB, vol. 10, no. 3, 1 June 2006 (2006-06-01), pages 282-289, XP025155097 ISSN: 1367-5931 [retrieved on 2006-06-01]

### SEQUENCE LISTING

<110> Santaris Pharma A/S
<120> ANTIDOTE OLIGOMERS
<130> 1058WO
<150> US61/077096
   <151> 2008-06-30
<150> US61/146326
   <151> 2009-01-22
<160> 18
<170> PatentIn version 3.5
<210> 1
   <211> 13
   <212> DNA
   <213> Artificial
<220>
   <223> LNA/DNA Oligomer
<400> 1
   gcattggtat tca 13
<210> 2
   <211> 13
   <212> DNA
   <213> Artificial
<220>
   <223> LNA/DNA Oligomer
<400> 2
   cgtaaccata agt 13
<210> 3
   <211> 16
   <212> DNA
   <213> Artificial
<220>
   <223> LNA/DNA Oligomer
<400> 3
   gtgtgtgtat ttccca 16
<210> 4
   <211> 16
   <212> DNA
   <213> Artificial
<220>
   <223> LNA/DNA Oligomer
<400> 4
   cacacacata aagggt 16
<210> 5
   <211> 16
   <212> DNA
   <213> Artificial
<220>
   <223> LNA/DNA Oligomer
<400> 5
   tggcaagcat cctgta 16
<210> 6
   <211> 16
   <212> DNA
   <213> Artificial
<220>
   <223> LNA/DNA Oligomer
<400> 6
   accgttcgta ggacat 16
<210> 7
   <211> 12
   <212> DNA
   <213> Artificial
<220>
   <223> LNA/DNA Oligomer
<400> 7
   gcaagcatcc tg 12
<210> 8
   <211> 12
   <212> DNA
   <213> Artificial
<220>
   <223> LNA/DNA Oligomer
<400> 8
   cgttcgtagg ac 12
<210> 9
   <211> 15
   <212> DNA
   <213> Artificial
<220>
   <223> LNA/DNA Oligomer
<400> 9
   ccattgtcac actcc 15
<210> 10
   <211> 15
   <212> DNA
   <213> Artificial
<220>
   <223> LNA/DNA Oligomer
<400> 10
   ggtaacagtg tgagg 15
<210> 11
   <211> 15
   <212> DNA
   <213> Artificial
<220>
   <223> LNA/DNA Oligomer
<400> 11
   tgcatggatt tgcac 15
<210> 12
   <211> 15
   <212> DNA
   <213> Artificial
<220>
   <223> LNA/DNA Oligomer
<400> 12
   acgtacctaa acgtg 15
<210> 13
   <211> 15
   <212> DNA
   <213> Artificial
<220>
   <223> LNA/DNA Oligomer
<400> 13
   tcacgattag catta 15
<210> 14
   <211> 15
   <212> DNA
   <213> Artificial
<220>
   <223> LNA/DNA Oligomer
<400> 14
   agtgctaatc gtaat 15
<210> 15
   <211> 15
   <212> DNA
   <213> Artificial
<220>
   <223> LNA/DNA Oligomer
<400> 15
   tcagtctgat aagct 15
<210> 16
   <211> 15
   <212> DNA
   <213> Artificial
<220>
   <223> LNA/DNA Oligomer
<400> 16
   agtcagacta ttcga 15
<210> 17
   <211> 15
   <212> DNA
   <213> Artificial
<220>
   <223> LNA/DNA Oligomer
<400> 17
   gtgccgttcg ttctt 15
<210> 18
   <211> 15
   <212> DNA
   <213> Artificial
<220>
   <223> LNA/DNA Oligomer
<400> 18
   cacggcaagc aagaa 15

## Description

### FIELD OF INVENTION

The present invention relates to antidote oligomeric compounds (oligomers), which target nucleotide based therapeutics *in vivo,* thereby providing a method of controlling the bioavailability and therefore the therapeutic activity and/or side effects of oligonucleotide based therapeutics *in vivo.*

### RELATED CASES

This application claims the benefit under 35 U.S.C. § 119(e) of U.S. Provisional Applications Serial Nos., US61/077096, filed 30th June 2008 and US61/146326 filed 22nd January 2009.

### BACKGROUND

Antidotes to therapeutic molecules are useful in treatments where there is a risk of the patient suffering deleterious side effects from the treatment. The availability of an antidote allows the physician to administer therapeutic agents at a level which has the optimal efficacy in treatment the medical condition, with the knowledge that if/when the patient exhibits deleterious side effects of the treatment, the antidote can be administered, thereby effectively deactivating the therapeutic agent *in vivo.*

WO 02/096926 discloses nucleic acid ligands (e.g. aptamers) which bind to for example protein targets due to its secondary and tertiary structure and how these aptamers can be modulated by a modulator molecule, such as an oligomer.

Jepsen et al 2004 Current opinion in drug discovery and development VI 7 page 188-194 describes various designs of the therapeutic oligomer.

WO2008/066621A discloses aptamer based antiplatelet agents and antidotes to antiplatelet agents and to methods of using such antidotes to reverse aptamer-induced platelet inhibition. The antidote is, apparently an oligonucleotide which may be between 10-15 nucleotides in length, although no designs of the oligonucleotide appear to have been disclosed.

WO2009/061841 refers to antidotes to antisense compounds. This application published after the priority claimed by the present application.

There remains therefore a need for antidotes for oligonucleotide therapeutics.

### SUMMARY OF INVENTION

The invention provides a kit, for therapeutic use, comprising a first oligomer in a first compartment and a second oligomer in a second compartment, wherein the first oligomer is a therapeutic oligomer which has a contiguous nucleotide sequence complementary or essentially complementary to a target nucleic acid naturally present in the subject and the first oligomer comprise one or more nucleotide analogues selected from locked nucleic acid (LNA), 2'-O-alkyl-RNA, 2'-OMe-RNA, 2'-amino-DNA, 2'-fluoro-DNA, 2'-MOE-RNA; and wherein the second oligomer is of between 6 and 30 contiguous nucleotides in length; wherein the sequence of contiguous nucleotides of said second oligomer is either i) completely complementary to a sequence of contiguous nucleotides present in said first oligomer over the length of the shorter of the two oligomers, or ii) comprises no more than a single mismatch with the complement of a sequence of contiguous nucleotides present in said first oligomer over the length of the shorter of the two oligomers and said second oligomer comprise one or more nucleotide analogues selected from LNA, 2'-O-alkyl-RNA, 2'-OMe-RNA, 2'-amino-DNA, 2'-fluoro-DNA, 2'-MOE-RNA and is designed so that the duplex between the first oligomer and the complementary second oligomer results in the formation of at last one nucleotide analogue base pair and the second oligomer does not comprise a contiguous sequence of more than 3 DNA units; wherein said first and said second oligomers are isolated from one another.

The first oligomer may, in some embodiments, be in the form of a oligomer which is either i) fully complementary to a sub-sequence of contiguous nucleotides present in a mammalian RNA target, such as a miRNA or mRNA, or ii) comprises no more than a single mismatch with the complement of a sub-sequence of contiguous nucleotides present in said RNA target.

The RNA target (of the first oligomer) may be associated with a medical condition or disease. In some embodiments, the RNA target may be a microRNA or a mRNA, for example. The first oligomer may, therefore be, for example, an antimiR, a microRNA mimic, a microRNA blockmir, or an antisense oligomer.

However, it is also considered that the first oligomer may be in the form of a siRNA (*i.e.* the sense or antisense strand of said siRNA). In this respect the antidote may target the passenger strand of the siRNA, thereby reducing or eliminating off target effects caused by the passenger strand.

In some embodiments, the second oligomer is in the form of a mixmer or totalmer, comprising one or more nucleotide analogues selected from LNA, 2'-O-alkyl-RNA, 2'-OMe-RNA, 2'-amino-DNA, 2'-fluoro-DNA, 2'-MOE-RNA and is designed so that the duplex between the first oligomer and the complementary second oligomer results in the formation of at last one nucleotide analogue base pair; and the second oligomer does not comprise a contiguous sequence of more than 3 DNA units. In preferred embodiment the second oligomer comprise one or more LNA nucleotide analogues, or consisting of LNA nucleotide analogues.

The application further describes a method of treatment comprising the sequential steps of:
a) administering a therapeutically effective amount of a first oligomer according to the invention to a subject (typically a patient),
b) either; i) allowing sufficient time to pass to allow for the first oligomer to provide a beneficial therapeutic effect, or ii) monitoring the subject to identify one or more adverse responses to the administration of the first oligomer to the subject,
c) administering a second oligomer according to the invention to the subject.
wherein the amount of the second oligomer administered is sufficient to reduce the bioavailability of the first oligomer, and/or the severity of the adverse response(s).

The application describesa method of treatment comprising the sequential steps of:
a) administering a therapeutically effective amount of a first oligomer to a patient, wherein said first oligomer is has a contiguous nucleotide sequence which is either i) fully complementary to a sub-sequence of contiguous nucleotides present in a mammalian RNA target or ii) comprises no more than a single mismatch with the complement of a sub-sequence of contiguous nucleotides present in said RNA target
b) either; i) allowing sufficient time to pass to allow for the first oligomer to down-regulate the expression the RNA target in said patient, or ii) monitoring the subject to identify one or more adverse responses to the administration of the first oligomer to the patient,
c) administering a second oligomer according to the invention to the patient,
wherein the amount of the second oligomer administered is sufficient to reduce the ability of the first oligomer to modulate the expression of the RNA target in said patient, such as up-regulate or down-regulate the expression of the RNA target in said patient.

The application describes a method for the deactivation of a first oligomer *in vivo* in a subject, said method comprising administering a second oligomer to a subject who has previously been administered the first oligomer, wherein said first and second oligomers are as according to the invention.

The application also describes designs for the second 'antidote' oligomer, such as the mimxer and totalmer oligomers described herein. The invention also provides antidote oligomers as described herein, and for their use in medicine.

The invention provides for the use of a second oligomer according to the invention for the manufacture of an antidote composition for the deactivation of a first oligomer *in vivo* in a subject.

The application describes an oligomer as defined according to the second oligomer of the invention for the inactivation of a first oligomer *in vivo* in a subject.

The application further describes a method for reducing the effective amount of a first oligomer in a cell or an organism, comprising administering a second oligomer (such as in the form of a pharmaceutical composition) according to the invention to a subject. Reducing the effective amount in this context refers to the reduction of the level of functional first oligomer present in the cell subject or a tissue or cell thereof.

### BRIEF DESCRIPTION OF FIGURES

**Figure 1****:** The 'antidote' approach. In this representation the 'antidote' second oligomer blocks the activity of oligonucleotide based drugs stoichiometrically.
   and in a sequence specific manner, The inclusion of LNA into the antidote oligomer is preferred as it results in highly efficient inactivation of the first oligomer.

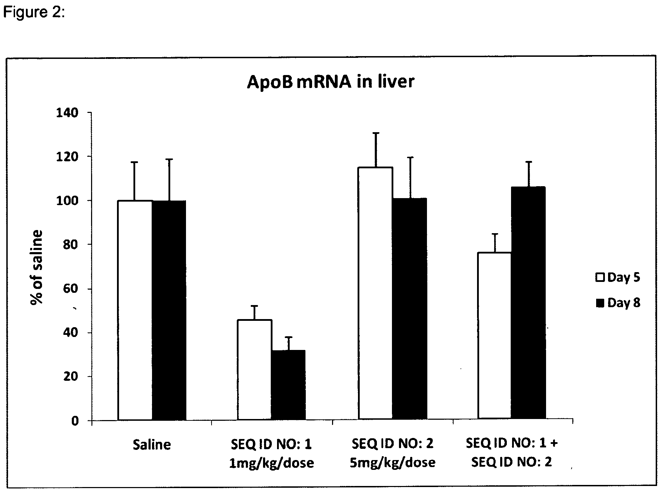

**Figure 2****:** In *vivo* experiment - ApoB mRNA in liver.

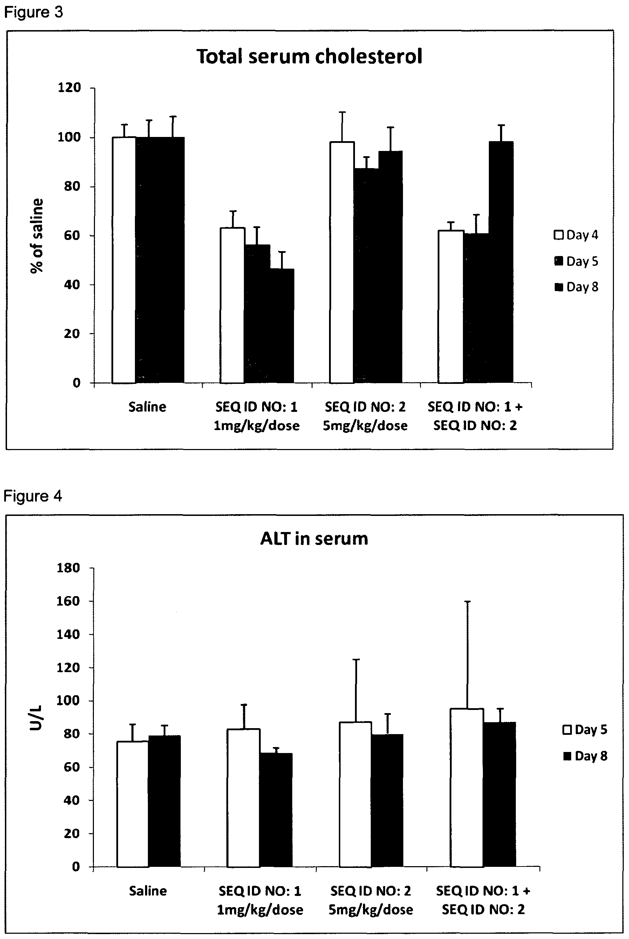

**Figure 3****:** In *vivo* experiment - Total serum cholesterol.
**Figure 4****:** In *vivo* experiment - ALT in serum.
**Figure 5****:** ISIS Pharmaceuticals Product Pipeline see -
   http://www.isispharm.com/product_pipeline.html

### DETAILED DESCRIPTION OF INVENTION

### Oligomers

The present invention employs oligomeric compounds (referred herein as the second oligomer, or antidote oligomer herein), for use in modulating the function of one or more therapeutic oligomers (referred herein as the first oligomer), *in vivo* in a subject, such as a human being. The term "oligomer" in the context of the present invention, refers to a molecule formed by covalent linkage of two or more nucleotides (*i.e.* an oligonucleotide).

The antidote oligomers may be a single stranded molecule, and, in some embodiments, do not comprise short regions of, for example, at least 3, 4 or 5 contiguous nucleotides, which are complementary to equivalent regions within the same oligomer (*i.e.* duplexes) - in this regards, the antidote oligomer may, in some embodiments, not be (essentially) double stranded. In some embodiments, the antidote oligomer is essentially not double stranded, such as is not a siRNA or does not form part of an siRNA. In some embodiments, the antidote oligomers do not comprise RNA (units). It will be recognised that the first oligomer (therapeutic oligomer), may be a single stranded molecule, and, in some embodiments, do not comprise short regions of, for example, at least 3, 4 or 5 contiguous nucleotides, which are complementary to equivalent regions within the same oligomer (*i.e.* duplexes) - in this regards, the therapeutic oligomer may, in some embodiments, not be (essentially) double stranded. In some embodiments, the therapeutic oligomer is essentially not double stranded, such as is not a siRNA. In some embodiments, the therapeutic oligomer does not comprise RNA (units). However, in some embodiments, the first oligomer forms part of an siRNA and may comprise RNA units. In some embodiments, the oligomers may consist entirely of the contiguous nucleotide region.

The second oligomer specifically hybridises to the first oligomer due to complementarity between the respective contiguous nucleotide sequences of the first and second oligomers. Preferably the complementarity over the length of the shorter of the two oligomers is completely complementary, although it is recognised that there may be a single mismatch in some embodiments. When we refer to the complement herein we refer to the reverse complement. The respective contiguous nucleotide sequences of the first and second oligimer may therefore be the reverse complement to each other. In some embodiments, the first contiguous nucleotide sequence of the first oligomer is the reverse complement of a sub-sequence of a RNA target, such as a mRNA, and miRNA or a strand of an siRNA, such as the passenger or antisense strand - it is typical that the contiguous nucleotide sequence of the first oligomer is perfectly complementary to the sub-sequence of the RNA target (the therapeutic target), although it is recognised that there may be a single mismatch in some embodiments.

The terms "reverse complement", "reverse complementary" and "reverse complementarity" as used herein are interchangeable with the terms "complement", "complementary" and "complementarity". As used herein, the terms "homologous" and "homology" are interchangeable with the terms "identity" and "identical".

Suitably, the first and second oligomer are capable of forming a duplex where the contiguous nucleotide sequences hybridise between the corresponding nucleotide sequences. As described herein, in some embodiments, for example when the second oligomer may be shorter than the first oligomer, the duplex formed between the first and second oligomer can, in some embodiments, result in overhang(s) between the two oligomers. The result of the hybridisation is that the first oligomer becomes unavailable for hybridisation with its therapeutic target *in vivo,* effectively decreasing the bioavailability of the first oligomer. In effect the first oligomer is deactivated *in vivo.* It should however be recognised that, due to the formation of stable duplexes between the first and second oligomer, the cellular abundance of the first oligomer may not actually decrease. Indeed, the use of second oligomers which comprise a high proportion of high affinity nucleotide analogues, such as mixmers or totalmers, have been found to be highly effective in titrating out nucleic acids *in vivo,* effectively deactivating the target nucleic acid by the formation of a remarkable stable duplex. The reduced bioavailability of the first oligomer can easily be assayed using hybridisation techniques as both the total reduction in first oligomer level, or the titration of the first oligomer by the formation of a stable duplex with the second oligomer will decrease the availability of the first oligomer to hybridise with a complementary detection probe, such as a qPCR or radio labelled detection probe. Alternatively, as illustrated by the examples the reduced bioavailabilty can be determined by monitoring the alleviation of the modulation of the therapeutic target RNA due to the first oligomer, such as up or down-regulation of the therapeutic target.

### The First Oligomer

The first oligomer is suitably an oligomer of length between 8 and 50 nucleotides in length, which comprises or consists of a contiguous nucleotide sequence, which typically is of at least 8 nucleotides in length. The contiguous nucleotide sequence of the first oligomer may be between 8 and 50 nucleotides in length. In some embodiments the length of the first oligomer, or the contiguous nucleotide sequence thereof is between 10 and 30 nucleotides, such as between 12 and 25 nucleotides, such as 13, 14, 15, 16, 17, 18, 19, 20 ,21, 22, 23 or 24 nucleotides in length, or between 10 and 18 nucleotides, or between 10 and 16 nucleotides, such as 10, 11, 12, 13, 14, 15 and 16 nucleotides, or between 12 and 16, or between 12 and 14 nucleotides.

The first oligomer is for *in vivo* use in a subject, such as an individual mammal, preferably a human. The subject is typically a patient who is suffering from or is likely to suffer from a medical disease or condition. Suitably the first oligomer is administered to the subject for the purpose of treating the medical disease or condition. The first oligomer may therefore be a therapeutic oligomer and the terms first oligomer and therapeutic oligomer may therefore be used herein to refer to the first oligomer.

The first oligomer may consist or comprise of a contiguous nucleotide sequence which is fully complementary to a sub-sequence of the target, such as a human mRNA or microRNA target, or, when base paired to the target RNA, in some embodiments comprise only a single mismatch to the sub-sequence of the mRNA target.

The first oligomer, may, for example, be in the form of an antisense oligonucleotide, an antimiR, a microRNA blockmir (*i.e.* an oligo which targets the microRNA regonition site on an mRNA target, such as the anti-seed region), an aptamer, a spiegelmer or a siRNA, such as the sense or antisense strand of the siRNA. In a preferable embodiment, the first oligomer is an antisense oligonucleotide which targets (*i.e.* is complementary or essentially complementary to a sequence present in an mRNA or microRNA.

Therefore, the therapeutic 'first' oligomer may, in various embodiments, target either i) target nucleic acids, for example mRNAs (antisense oligomers) or microRNA (antimiRs) or ii) target proteins in the subject (aptamers and spiegelmers).

The first oligomer may in some embodiments comprise or consist of both naturally occurring nucleotides and non-naturally occurring nucleotides.

In some embodiments, the first oligomer may be in the form of a gapmer, a blockmer, a headmer, a tailmer, or a mixmer. In some embodiments, the first oligomer is a gapmer oligomer or a 'shortmer' oligomer. In some embodiments the first oligomer is a antimir or blockmir, and as such, may, in some embodiments be a mixmer or totalmer.

In some embodiments, the first oligomer or contiguous nucleotide sequence thereof consists of a contiguous sequence of nucleotide analogues, such as affinity enhancing nucleotide analogues - referred to herein is as a 'totalmer'.

### Antisense oligomers

In some embodiments, the first oligomer is an antisense oligomer (also referred to as an antisense oligonucleoitde) of between 8 and 30 nucleotides in length. The nucleotides may be naturally occurring nucleotides, such as DNA or RNA, or non-naturally occurring nucleotides, *i.e.* nucleotide analogues.

The antisense oligomer is perfectly complementary or essentially complementary (such as comprises no more than a single mismatch) to a target nucleic acid naturally present in the subject, such as a target RNA or subsequence thereof, such as an mRNA or microRNA sequence.

In some embodiments the antisense oligomer is in the form of a gapmer, a headmer or a tailmer. Gapmer designs are typically used to target mRNA targets. However, it is also recognised that the antisense oligomer may operate via non RNAse(H) mechanisms.The antisense oligomer may, in some embodiments, be in the form of a mixmer or totamer.

### RNAse H recruitment

In some embodiments, an oligomer functions via non-RNase-mediated degradation of a target mRNA, such as by steric hindrance of translation, or other mechanisms; however, in various embodiments, oligomers of the invention are capable of recruiting an endo-ribonuclease (RNase), such as RNase H.

Typically, the oligomer, comprises a region of at least 6, such as at least 7 contiguous monomers, such as at least 8 or at least 9 contiguous monomers, including 7, 8, 9, 10, 11, 12, 13, 14, 15 or 16 contiguous monomers, which, when forming a duplex with the target region of the target RNA, is capable of recruiting RNase. The region of the oligomer which is capable of recruiting RNAse may be region B, as referred to in the context of a gapmer as described herein. In some embodiments, the region of the oligomer which is capable of recruiting RNAse, such as region B, consists of 10, 11, 12, 13, 14, 15, 16, 17, 18, 19 or 20 monomers.

EP 1 222 309 provides *in vitro* methods for determining RNaseH activity, which may be used to determine the ability of the oligomers of the invention to recruit RNaseH. An oligomer is deemed capable of recruiting RNaseH if, when contacted with the complementary region of the RNA target, it has an initial rate, as measured in pmol/l/min, of at least 1%, such as at least 5%, such as at least 10% or more than 20% of the initial rate determined using an oligonucleotide having the same base sequence but containing only DNA monomers, with no 2' substitutions, with phosphorothioate linkage groups between all monomers in the oligonucleotide, using the methodology provided by Examples 91 - 95 of EP 1 222 309.

In some embodiments, an oligomer is deemed essentially incapable of recruiting RNaseH if, when contacted with the complementary target region of the RNA target, and RNaseH, the RNaseH initial rate, as measured in pmol/l/min, is less than 1%, such as less than 5%, such as less than 10% or less than 20% of the initial rate determined using an oligonucleotide having the same base sequence, but containing only DNA monomers, with no 2' substitutions, with phosphorothioate linkage groups between all monomers in the oligonucleotide, using the methodology provided by Examples 91 - 95 of EP 1 222 309.

In other embodiments, an oligomer is deemed capable of recruiting RNaseH if, when contacted with the complementary target region of the RNA target, and RNaseH, the RNaseH initial rate, as measured in pmol/l/min, is at least 20%, such as at least 40%, such as at least 60%, such as at least 80% of the initial rate determined using an oligonucleotide having the same base sequence, but containing only DNA monomers, with no 2' substitutions, with phosphorothioate linkage groups between all monomers in the oligonucleotide, using the methodology provided by Examples 91 - 95 of EP 1 222 309.

Typically, the region of the oligomer which forms the duplex with the complementary target region of the target RNA and is capable of recruiting RNase contains DNA monomers and LNA monomers and forms a DNA/RNA-like duplex with the target region. The LNA monomers are preferably in the alpha-L configuration, particularly preferred being alpha-L-oxy LNA.

In various embodiments, the oligomer of the invention comprises both nucleosides and nucleoside analogues, and is in the form of a gapmer, a headmer or a mixmer.

### Gapmer Design

A gapmer oligomer is an oligomer which comprises a contiguous stretch of nucleotides which is capable of recruiting an RNAse, such as RNAseH, such as a region of at least 6 or 7 DNA nucleotides, referred to herein in as region B, wherein region B is flanked both 5' and 3' by regions of affinity enhancing nucleotide analogues, such as between 1 - 6 nucleotide analogues 5' and 3' to the contiguous stretch of nucleotides which is capable of recruiting RNAse - these regions are referred to as regions A and C respectively.

In some embodiments, the nucleotides which are capable of recruiting RNAse are selected from the group consisting of DNA nucleotides, alpha-L-LNA nucleotides, C4' alkylayted DNA.(see WO 2009/090182 ), and UNA nucleotides (see Fluiter et al., Mol. Biosyst., 2009, 10, 1039). In some embodiments, region B consists of a contiguous length of at least 6 or 7 DNA nucleotides, or nucleotides selected from the group consisting of DNA and alpha-L-LNA.

Preferably the gapmer comprises a (poly)nucleotide sequence of formula (5' to 3'), A-B-C, or optionally A-B-C-D or D-A-B-C, wherein; region A (5' region) consists or comprises of at least one nucleotide analogue, such as at least one LNA unit, such as between 1-6 nucleotide analogues, such as LNA units, and; region B consists or comprises of at least five consecutive nucleotides which are capable of recruiting RNAse (when formed in a duplex with a complementary RNA molecule, such as the mRNA target), such as DNA nucleotides, and; region C (3'region) consists or comprises of at least one nucleotide analogue, such as at least one LNA unit, such as between 1-6 nucleotide analogues, such as LNA units, and; region D, when present consists or comprises of 1, 2 or 3 nucleotide units, such as DNA nucleotides.

In various embodiments, region A consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units; and/or region C consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units.

In various embodiments B consists or comprises of 5, 6, 7, 8, 9, 10, 11 or 12 consecutive nucleotides which are capable of recruiting RNAse, or between 6-10, or between 7-9, such as 8 consecutive nucleotides which are capable of recruiting RNAse. In various embodiments region B consists or comprises at least one DNA nucleotide unit, such as 1-12 DNA units, preferably between 4-12 DNA units, more preferably between 6-10 DNA units, such as between 7-10 DNA units, most preferably 8, 9 or 10 DNA units.

In various embodiments region A consist of 3 or 4 nucleotide analogues, such as LNA, region B consists of 7, 8, 9 or 10 DNA units, and region C consists of 3 or 4 nucleotide analogues, such as LNA. Such designs include (A-B-C) 3-10-3, 3-10-4, 4-10-3, 3-9-3, 3-9-4, 4-9-3, 3-8-3, 3-8-4, 4-8-3, 3-7-3, 3-7-4, 4-7-3, and may further include region D, which may have one or 2 nucleotide units, such as DNA units.

Further gapmer designs are disclosed in WO2004/046160.

US provisional application, 60/977409, refers to 'shortmer' gapmer oligomers, which, in various embodiments may be the gapmer oligomer according to the present invention.

In various embodiments the oligomer is consisting of a contiguous nucleotide sequence of a total of 10, 11, 12, 13 or 14 nucleotide units, wherein the contiguous nucleotide sequence is of formula (5' - 3'), A-B-C, or optionally A-B-C-D or D-A-B-C, wherein; A consists of 1, 2 or 3 nucleotide analogue units, such as LNA units; B consists of 7, 8 or 9 contiguous nucleotide units which are capable of recruiting RNAse when formed in a duplex with a complementary RNA molecule (such as a mRNA target); and C consists of 1, 2 or 3 nucleotide analogue units, such as LNA units. When present, D consists of a single DNA unit.

In various embodiments A consists of 1 LNA unit. In various embodiments A consists of 2 LNA units. In various embodiments A consists of 3 LNA units. In various embodiments C consists of 1 LNA unit. In various embodiments C consists of 2 LNA units. In various embodiments C consists of 3 LNA units. In various embodiments B consists of 7 nucleotide units. In various embodiments B consists of 8 nucleotide units. In various embodiments B consists of 9 nucleotide units. In various embodiments B comprises of between 1 - 9 DNA units, such as 2, 3, 4, 5, 6, 7 or 8 DNA units. In various embodiments B consists of DNA units. In various embodiments B comprises of at least one LNA unit which is in the alpha-L configuration, such as 2, 3, 4, 5, 6, 7, 8 or 9 LNA units in the alpha-L-configuration. In various embodiments B comprises of at least one alpha-L-oxy LNA unit or wherein all the LNA units in the alpha-L- configuration are alpha-L-oxy LNA units. In various embodiments the number of nucleotides present in A-B-C are selected from the group consisting of (nucleotide analogue units - region B - nucleotide analogue units): 1-8-1, 1-8-2, 2-8-1, 2-8-2, 3-8-3, 2-8-3, 3-8-2, 4-8-1, 4-8-2, 1-8-4, 2-8-4, or; 1-9-1, 1-9-2, 2-9-1, 2-9-2, 2-9-3, 3-9-2, 1-9-3, 3-9-1, 4-9-1, 1-9-4, or; 1-10-1, 1-10-2, 2-10-1, 2-10-2, 1-10-3, 3-10-1. In various embodiments the number of nucleotides in A-B-C are selected from the group consisting of: 2-7-1, 1-7-2, 2-7-2, 3-7-3, 2-7-3, 3-7-2, 3-7-4, and 4-7-3. In various embodiments both A and C consists of two LNA units each, and B consists of 8 or 9 nucleotide units, preferably DNA units.

### Splice switching oligomers

In some embodiments, the antisense oligonucleotide is a splice switching oligomer - *i.e.* an oligomer which targets the pre-mRNA causing an alternative splicing of the pre-mRNA.

Targets for the splice switching oligomer may include TNF receptor, for example the SSO may be one or more of the TNFR SSOs disclosed in WO2007/058894, WO08051306 A1 and WO 2008/131807.

Splice switching oligomers are typically(essentially) not capable of recruiting RNaseH and as such gapmer, tailmer or headmer designs are generally not desirable. However, mixmer and totalmers designs are suitable designs for SSOs.

### mRNA Targets

The target of the first oligomer (antisense oligomer) may be a RNA of a gene which is associated with a disease or medical disorder - such as a mRNA, the down-regulation of the RNA target providing a therapeutic benefit.

In some embodiments the first oligomer targets the ApoB100 mRNA, such as the oligomers disclosed in WO2007/031081 or WO2008/113830.

In some embodiments the first oligomer targets the PCSK9 mRNA, such as the oligomers disclosed in WO2008/043753.

In some embodiments the first oligomer targets the Hif1 alpha mRNA, such as a oligomer selected from the LNA oligomers disclosed in WO03/085110 or WO2006/050734, WO2009/043759, or WO2008/113832.

In some embodiments the first oligomer targets the survivin mRNA, such as the oligomers disclosed in WO2004/069991 or WO2006/050732.

In some embodiments the first oligomer targets the Bcl2 mRNA, such as the oligomers disclosed in WO2005/061710 or WO2009/071681.

In some embodiments, the first oligomer targets the Mcl1 mRNA, such as the oligomers disclosed in WO2009/071680.

In some embodiments, the first oligomer targets the FABP4 mRNA, such as the oligomers disclosed in WO2009/027527.

In some embodiments, the first oligomer targets the Pl3 Kinase mRNA, such as the oligomers disclosed in WO2009/071082.

In some embodiments, the first oligomer targets the androgen receptor mRNA, such as the oligomers disclosed in WO2009/068033.

In some embodiments, the first oligomer targets the TNFR-alpha mRNA, such as the oligomers disclosed in WO2007/058894 or WO2008/131807 (both disclose SSOs targeting TNFR1 and TNFR2).

In some embodiments the first oligomer targets the beta-catenin mRNA, such as the oligomers disclosed in WO2008/132234.

In some embodiments the first oligomer targets the EGFR/HER3 mRNA, such as the oligomers disclosed in WO2008/138904.

In some embodiments the first oligomer is selected from the oligomers disclosed in WO2008/113832.

In some embodiments the medical disorder is selected from the group consisting of cardiovascular disorders (such as hyperlipidepia), metabolic disorders (such as diabetes), hyperproliferative disorders (such as cancer), inflammatory disorders (such as multiple scleorisis, ulcerative colitis, arthritis, inflammatory bowel disease, and asthma), viral diseases (such as hepatitis C, HIV infection, CMV), ocular disease.

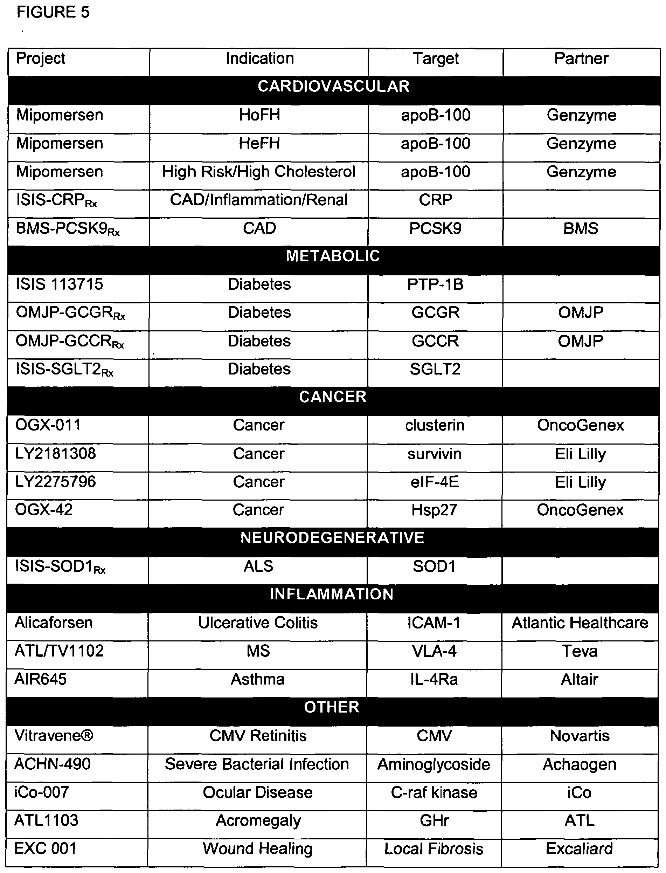

The first oligomer may be an oligomer developed by ISIS Pharmaceutical Inc, such as an oligomer selected from the table shown in Figure 5, or an oligomer which targets an mRNA listed in the following table or a first oligomer which is for use in the medical indications listed Figure 5. Mipomersen is ISIS 301012, as disclosed in US 7,511,131. ISIS 353512 is disclosed in WO2005/005599. Graham et al., Journal of Lipid Research, Vol. 48, 763-767, April 2007 and WO08066776 discloses PCSK9 antisense compounds, including ISIS 394814. ISIS 113715 is disclosed in WO06044531. ISIS 325568 is disclosed in US2006063730 and WO06034348. ISIS 377131 is disclosed in US2006063730. ISIS 388626 is disclosed in US2008015162. OGX-011 is disclosed in Schmitz, Current Opinion in Molecular Therapeutics 2006 8(6):547-554. LY2181308, also referred to as ISIS23722, is disclosed in US2002137708A. Ly2275796 is the oligo with SEQ ID No 40 of US 7,425,544. OGX-427 is disclosed in Kamada et al., Mol. Cancer. Therapeutics 2007 6 299. Alicaforsen, also known as ISIS 2302 as disclosed in WO9405333, ALT/TV1102 is disclosed as SEQ ID NO 81 in EP 1 123 414. ISIS 369645 is disclosed in, e.g., Karras, J. G. et al. (2007) Am J Respir Cell Mol Biol. 36(3):276-86). ISIS-14803 is an HCV targeting antisense oligonucleotide being developed by Isis and Merck, and is disclosed in WO9929350. iCo-007, also known as ISIS 13650, is disclosed in US2003083280.
ISIS 333611 is disclosed in WO2005/040180. ALT1103 - WO2004/078922. mRNA selected from the group consisting of ApoB-100, CRP, PCSK9, PTP-1 B, GCGR, GCCR, SGLT2, clusterin, surviving, eiF-4E, Hsp27, ICAM-1, VLA-4, IL-4Ralpha, C-ref kinase, SOD1, and GHr.

### Gapmer oligomers

Gapmer oligomers are a preferred design of oligomer for the targeting of mRNA targets, and as such, in some embodiments, the first oligomer is a gapmer oligomer.

A gapmer oligomer is an oligomer which comprises a contiguous stretch of nucleotides which is capable of recruiting RNAse, such as RNAseH, such as a region of at least 6 or 7 DNA nucleotides, referred to herein in as region B (B), wherein region B is flanked both 5' and 3' by regions of affinity enhancing nucleotide analogues, such as between 1 - 6 nucleotide analogues 5' and 3' to the contiguous stretch of nucleotides which is capable of recruiting RNAse - these regions are referred to as region A (A) and region C (C) respectively. In various embodiments, the first oligomer is a gapmer oligomer which comprises a contiguous nucleotide sequences A-B-C(-D) or (D-)A-B-C, wherein A comprises or consists of between 1 and 6 consecutive nucleotide analogues; B comprises or consists of between 6 and 14 consecutive nucleotides; C comprises of between 1 and 6 consecutive nucleotide analogues; and D, which is optional, may comprise of 1, 2 or 3 natural nucleotides, such as DNA nucleotides.

In some embodiments, the monomers which are capable of recruiting RNAse are selected from the group consisting of DNA monomers, alpha-L-LNA monomer, C4' alkylayted DNA monomers (see Vester et al., Bioorg. Med. Chem. Lett. 18 (2008) 22-96 - 2300), and UNA nucleotides (see Fluiter et al., Mol. Biosyst., 2009, 10, 1039).

Preferably the gapmer comprises a (poly)nucleotide sequence of formula (5' to 3'), A-B-C, or optionally A-B-C-D or D-A-B-C, wherein; region A (5' region) consists or comprises of at least one nucleotide analogue, such as at least one LNA unit, such as between 1-6 nucleotide analogues, such as LNA units, and; region B consists or comprises of at least five consecutive nucleotides which are capable of recruiting RNAse (when formed in a duplex with a complementary RNA molecule, such as the mRNA target), such as DNA nucleotides, and; region C (3'region) consists or comprises of at least one nucleotide analogue, such as at least one LNA unit, such as between 1-6 nucleotide analogues, such as LNA units, and; region D, when present consists or comprises of 1, 2 or 3 nucleotide units, such as DNA nucleotides.

In some embodiments, region A consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units; and/or region C consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units. In some embodiments, in region A the two or more nucleoside analogues are contiguous (consecutive) nucleoside analogues. In various embodiments, in region C the two or more nucleoside analogues are contiguous (consecutive) nucleoside analogues.

In some embodiments B consists or comprises of 5, 6, 7, 8, 9, 10, 11 or 12 consecutive nucleotides which are capable of recruiting RNAse, or between 6-10, or between 7-9, such as 8 consecutive nucleotides which are capable of recruiting RNAse. In some embodiments region B consists or comprises at least one DNA nucleotide unit, such as 1-12 DNA units, preferably between 4-12 DNA units, more preferably between 6-10 DNA units, such as between 7-10 DNA units, most preferably 8, 9 or 10 DNA units.

In some embodiments region A consist of 3 or 4 nucleotide analogues, such as LNA, region B consists of 7, 8, 9 or 10 DNA units, and region C consists of 3 or 4 nucleotide analogues, such as LNA. Such designs include (A-B-C) 3-10-3, 3-10-4, 4-10-3, 3-9-3, 3-9-4, 4-9-3, 3-8-3, 3-8-4, 4-8-3, 3-7-3, 3-7-4, 4-7-3, and may further include region D, which may have one or 2 nucleotide units, such as DNA units.

Further gapmer designs are disclosed in WO2004/046160. US provisional application, WO2008/113832, refers to 'shortmer' gapmer oligomers, which, in some embodiments may be the gapmer oligomer according to the present invention.

In some embodiments the oligomer is consisting of a contiguous nucleotide sequence of a total of 10, 11, 12, 13 or 14 nucleotide units, wherein the contiguous nucleotide sequence is of formula (5' - 3'), A-B-C, or optionally A-B-C-D or D-A-B-C, wherein; A consists of 1, 2 or 3 nucleotide analogue units, such as LNA units; B consists of 7, 8 or 9 contiguous nucleotide units which are capable of recruiting RNAse when formed in a duplex with a complementary RNA molecule (such as a mRNA target); and C consists of 1, 2 or 3 nucleotide analogue units, such as LNA units. When present, D consists of a single DNA unit.

In some embodiments A consists of 1 LNA unit. In some embodiments A consists of 2 LNA units. In some embodiments A consists of 3 LNA units. In some embodiments C consists of 1 LNA unit. In some embodiments C consists of 2 LNA units. In some embodiments C consists of 3 LNA units. In some embodiments B consists of 7 nucleotide units. In some embodiments B consists of 8 nucleotide units. In some embodiments B consists of 9 nucleotide units. In some embodiments B comprises of between 1 - 9 DNA units, such as 2, 3, 4, 5, 6, 7 or 8 DNA units. In some embodiments B consists of DNA units. In some embodiments B comprises of at least one LNA unit which is in the alpha-L configuration, such as 2, 3, 4, 5, 6, 7, 8 or 9 LNA units in the alpha-L-configuration. In some embodiments B comprises of at least one alpha-L-oxy LNA unit or wherein all the LNA units in the alpha-L- configuration are alpha-L-oxy LNA units. In some embodiments the number of nucleotides present in A-B-C are selected from the group consisting of (nucleotide analogue units - region B - nucleotide analogue units): 1-8-1, 1-8-2, 2-8-1, 2-8-2, 3-8-3, 2-8-3, 3-8-2, 4-8-1, 4-8-2, 1-8-4, 2-8-4, or;1-9-1, 1-9-2, 2-9-1, 2-9-2, 2-9-3, 3-9-2, 1-9-3, 3-9-1, , 3-9-3, 4-9-1, 1-9-4, or; 1-10-1, 1-10-2, 2-10-1, 2-10-2, 1-10-3, 3-10-1, 2-10-3, 3-10-2, or 3-10-3.. In some embodiments the number of nucleotides in A-B-C are selected from the group consisting of: 2-7-1, 1-7-2, 2-7-2, 3-7-3, 2-7-3, 3-7-2, 3-7-4, and 4-7-3. In some embodiments both A and C consists of two LNA units each, and B consists of 8 or 9 nucleotide units, preferably DNA units.. In certain embodiments, each of regions A and C consists of three LNA monomers, and region B consists of 8 or 9 or 10 nucleoside monomers, preferably DNA monomers.

In some embodiments, the first oligomer is a gapmer which comprises of a gap (region B) of between 8 and 12, or 8 and 14, such as 9, 10, or 11 consecutive 2'-deoxynucleotides (DNA). In some embodiments, which may be the same or different, region B is positioned between wing segments (regions A and C) of 2' modified nucleotides such as 2'-MOE nucleotides, such as 3, 4, 5 or 6 2'modified nucleotides such as 2'-MOE nucleotides. In various embodiments, the nucleotide analogues may be 2' modified nucleotides, such as nucleotides which comprise a 2'-F, 2'-OCH₂ (2'-0Me) or a 2' -O(CH₂)₂-OCH₃ (2'-O-methoxyethyl or 2'-MOE) substituent group.

The cytosine residues of the first oligomer, such as the nucleotide analogues and/or nucleotides, may be methylated - such as comprise a 5-methyl.

The first oligomer may, in some embodiments, have at least one modified internucleoside linkage, as described herein, such as phosphorothioate linkages. In some embodiments, all the internucleoside linkages may be a phosphorothioate internucleoside linkages.

### Mixmers

The term 'mixmer' refers to oligomers which comprise both naturally and non-naturally occurring nucleotides, where, as opposed to gapmers, tailmers, headmers and blockmers, there is no contiguous sequence of more than 5 naturally occurring nucleotides, such as DNA units.

The first and/or second oligomer according to the invention maybe mixmers - indeed various mixmer designs are highly effective as first oligomers, particularly when targeting microRNA (antimiRs), microRNA binding sites on mRNAs (Blockmirs) or as splice switching oligomers (SSOs).

The second oligomer may, in some embodiments, also be a mixmer and indeed, due to the ability of mixmers to effectively and specifically bind to their target, the use of mixmers as second oligomers are considered to be particularly effective in decreasing the bioavailability of the first oligomer.

In some embodiments, the mixmer comprises or consists of a contiguous nucleotide sequence of repeating pattern of nucleotide analogue and naturally occurring nucleotides, or one type of nucleotide analogue and a second type of nucleotide analogues. The repeating pattern, may, for instance be every second or every third nucleotide is a nucleotide analogue, such as LNA, and the remaining nucleotides are naturally occurring nucleotides, such as DNA, or are a 2'substituted nucleotide analogue such as 2'MOE of 2'fluoro analogues as referred to herein, or, in some embodiments selected form the groups of nucleotide analogues referred to herein. It is recognised that the repeating pattern of nucleotide analogues, such as LNA units, may be combined with nucleotide analogues at fixed positions - e.g. at the 5' or 3' termini.

In some embodiments the first nucleotide of the first and/or second oligomer, counting from the 3' end, is a nucleotide analogue, such as an LNA nucleotide.

In some embodiments, which maybe the same or different, the second nucleotide of the first and or second oligomer, counting from the 3' end, is a nucleotide analogue, such as an LNA nucleotide.

In some embodiments, which maybe the same or different, the seventh and/or eighth nucleotide of the first and or second oligomer, counting from the 3' end, are nucleotide analogues, such as LNA nucleotides.

In some embodiments, which maybe the same or different, the ninth and/or the tenth nucleotides of the first and/or second oligomer, counting from the 3' end, are nucleotide analogues, such as LNA nucleotides.

In some embodiments, which maybe the same or different, the 5' terminal of the first and or second oligomer is a nucleotide analogue, such as an LNA nucleotide.

The above design features may, in some embodiments be incorporated into the mixmer design, such as antimiR mixmers.

In some embodiments, the mixmer does not comprise a region of more than 4 consecutive DNA nucleotide units or 3 consecutive DNA nucleotide units. In some embodiments, the mixmer does not comprise a region of more than 2 consecutive DNA nucleotide units.

In some embodiments, the mixmer comprises at least a region consisting of at least two consecutive nucleotide analogue units, such as at least two consecutive LNA units.

In some embodiments, the mixmer comprises at least a region consisting of at least three consecutive nucleotide analogue units, such as at least three consecutive LNA units.

In some embodiments, the mixmer of the invention does not comprise a region of more than 7 consecutive nucleotide analogue units, such as LNA units. In some embodiments, the mixmer of the invention does not comprise a region of more than 6 consecutive nucleotide analogue units, such as LNA units. In some embodiments, the mixmer of the invention does not comprise a region of more than 5 consecutive nucleotide analogue units, such as LNA units. In some embodiments, the mixmer of the invention does not comprise a region of more than 4 consecutive nucleotide analogue units, such as LNA units. In some embodiments, the mixmer of the invention does not comprise a region of more than 3 consecutive nucleotide analogue units, such as LNA units. In some embodiments, the mixmer of the invention does not comprise a region of more than 2 consecutive nucleotide analogue units, such as LNA units.

In the mixmer, such as antimiR or second oligomer embodiments, which refer to the modification of nucleotides in positions 3 to 8, counting from the 3' end, the LNA units may be replaced with other nucleotide anlogues, such as those referred to herein. "X" may, therefore be selected from the group consisting of 2'-O-alkyl-RNA unit, 2'-OMe-RNA unit, 2'-amino-DNA unit, 2'-fluoro-DNA unit, 2'-MOE-RNA unit, LNA unit, PNA unit, HNA unit, INA unit. "x" is preferably DNA or RNA, most preferably DNA.

In some embodiments, the mixmer, such as an antimiR mixmer, is modified in positions 3 to 8 - *i.e.* comprises at least one nucleotide analogue in positions 3 to 8, counting from the 3' end. The design of this sequence may be defined by the number of non-LNA units present or by the number of LNA units present. In some embodiments of the former, at least one, such as one, of the nucleotides in positions three to eight, counting from the 3' end, is a non-LNA unit. In some embodiments, at least two, such as two, of the nucleotides in positions three to eight, counting from the 3' end, are non-LNA units. In some embodiments, at least three, such as three, of the nucleotides in positions three to eight, counting from the 3' end, are non-LNA units. In some embodiments, at least four, such as four, of the nucleotides in positions three to eight, counting from the 3' end, are non-LNA units. In some embodiments, at least five, such as five, of the nucleotides in positions three to eight, counting from the 3' end, are non-LNA units. In some embodiments, all six nucleotides in positions three to eight, counting from the 3' end, are non-LNA units.

Alternatively defined, in some embodiments, the mixmer, such as an antimiR mixmer, according to the invention comprises at least one LNA unit in positions three to eight, counting from the 3' end. some embodiments, the the mixmer, such as an antimiR mixmer, comprises one LNA unit in positions three to eight, counting from the 3' end. The substitution pattern for the nucleotides in positions three to eight, counting from the 3' end, may be selected from the group consisting of Xxxxxx, xXxxxx, xxXxxx, xxxXxx, xxxxXx and xxxxxX, wherein "X" denotes an LNA unit and "x" denotes a non-LNA unit.

In some embodiments, the mixmer, such as an antimiR mixmer, comprises at least two LNA units in positions three to eight, counting from the 3' end. In some embodiments thereof, the mixmer comprises two LNA units in positions three to eight, counting from the 3' end. The substitution pattern for the nucleotides in positions three to eight, counting from the 3' end, may be selected from the group consisting of XXxxxx, XxXxxx, XxxXxx, XxxxXx, XxxxxX, xXXxxx, xXxXxx, xXxxXx, xXxxxX, xxXXxx, xxXxXx, xxXxxX, xxxXXx, xxxXxX and xxxxXX, wherein "X" denotes an LNA unit and "x" denotes a non-LNA unit. In an embodiment, the substitution pattern for the nucleotides in positions three to eight, counting from the 3' end, is selected from the group consisting of XxXxxx, XxxXxx, XxxxXx, XxxxxX, xXxXxx, xXxxXx, xXxxxX, xxXxXx, xxXxxX and xxxXxX, wherein "X" denotes an LNA unit and "x" denotes a non-LNA unit. In some embodiments, the substitution pattern for the nucleotides in positions three to eight, counting from the 3' end, is selected from the group consisting of xXxXxx, xXxxXx, xXxxxX, xxXxXx, xxXxxX and xxxXxX, wherein "X" denotes an LNA unit and "x" denotes a non-LNA unit. In some embodiments, the substitution pattern for the nucleotides in positions three to eight, counting from the 3' end, is selected from the group consisting of xXxXxx, xXxxXx and xxXxXx, wherein "X" denotes an LNA unit and "x" denotes a non-LNA unit. In some embodiments, the substitution pattern for the nucleotides in positions three to eight, counting from the 3' end, is xXxXxx, wherein "X" denotes an LNA unit and "x" denotes a non-LNA unit.

In some embodiments, the mixmer, such as an antimiR mixmer, comprises at least three LNA units in positions three to eight, counting from the 3' end. In an embodiment thereof, the mixmer comprises three LNA units in positions three to eight, counting from the 3' end. The substitution pattern for the nucleotides in positions three to eight, counting from the 3' end, may be selected from the group consisting of XXXxxx, xXXXxx, xxXXXx, xxxXXX, XXxXxx, XXxxXx, XXxxxX, xXXxXx, xXXxxX, xxXXxX, XxXXxx, XxxXXx, XxxxXX, xXxXXx, xXxxXX, xxXxXX, xXxXxX and XxXxXx, wherein "X" denotes an LNA unit and "x" denotes a non-LNA unit. In some embodiments, the substitution pattern for the nucleotides in positions three to eight, counting from the 3' end, is selected from the group consisting of XXxXxx, XXxxXx, XXxxxX, xXXxXx, xXXxxX, xxXXxX, XxXXxx, XxxXXx, XxxxXX, xXxXXx, xXxxXX, xxXxXX, xXxXxX and XxXxXx, wherein "X" denotes an LNA unit and "x" denotes a non-LNA unit. In some embodiments, the substitution pattern for the nucleotides in positions three to eight, counting from the 3' end, is selected from the group consisting of xXXxXx, xXXxxX, xxXXxX, xXxXXx, xXxxXX, xxXxXX and xXxXxX, wherein "X" denotes an LNA unit and "x" denotes a non-LNA unit. In some embodiments, the substitution pattern for the nucleotides in positions three to eight, counting from the 3' end, is xXxXxX or XxXxXx, wherein "X" denotes an LNA unit and "x" denotes a non-LNA unit. In some embodiments, the substitution pattern for the nucleotides in positions three to eight, counting from the 3' end, is xXxXxX, wherein "X" denotes an LNA unit and "x" denotes a non-LNA unit.

In some embodiments, the mixmer comprises at least four LNA units in positions three to eight, counting from the 3' end. In some embodiments thereof, the mixmer comprises four LNA units in positions three to eight, counting from the 3' end. The substitution pattern for the nucleotides in positions three to eight, counting from the 3' end, may be selected from the group consisting of xxXXXX, xXxXXX, xXXxXX, xXXXxX, xXXXXx, XxxXXX, XxXxXX, XxXXxX, XxXXXx, XXxxXX, XXxXxX, XXxXXx, XXXxxX, XXXxXx and XXXXxx, wherein "X" denotes an LNA unit and "x" denotes a non-LNA unit.

In some embodiments, the mixmer according to the present invention comprises at least five LNA units in positions three to eight, counting from the 3' end. In some embodiments thereof, the mixmercomprises five LNA units in positions three to eight, counting from the 3' end. The substitution pattern for the nucleotides in positions three to eight, counting from the 3' end, may be selected from the group consisting of xXXXXX, XxXXXX, XXxXXX, XXXxXX, XXXXxX and XXXXXx, wherein "X" denotes an LNA unit and "x" denotes a non-LNA unit.

In some embodiments, said non-LNA unit is another nucleotide analogue unit.

In some mixmer embodiments the substitution pattern for the nucleotides from position 11, counting from the 3' end, to the 5' end may include nucleotide analogue units (such as LNA) or it may not. In some embodiments, the mixmer comprises at least one nucleotide analogue unit (such as LNA), such as one nucleotide analogue unit, from position 11, counting from the 3' end, to the 5' end. In some embodiments, the mixmer comprises at least two nucleotide analogue units, such as LNA units, such as two nucleotide analogue units, from position 11, counting from the 3' end, to the 5' end.

In some embodiments which refer to the modification of nucleotides in the nucleotides from position 11 to the 5' end of the oligomer, the LNA units may be replaced with other nucleotide anlogues, such as those referred to herein. "X" may, therefore be selected from the group consisting of 2'-O-alkyl-RNA unit, 2'-OMe-RNA unit, 2'-amino-DNA unit, 2'-fluoro-DNA unit, LNA unit, PNA unit, HNA unit, INA unit. "x" is preferably DNA or RNA, most preferably DNA.

In some embodiments, the mixmer has the following substitution pattern, which is repeated from nucleotide eleven, counting from the 3' end, to the 5' end: xXxX or XxXx, wherein "X" denotes an LNA unit and "x" denotes a non-LNA unit. In another embodiment, the mixmer has the following substitution pattern, which is repeated from nucleotide eleven, counting from the 3' end, to the 5' end: XXxXxx, XXxxXx or XxXxxX, wherein "X" denotes an LNA unit and "x" denotes a non-LNA unit. In yet another embodiment, the mixmer has the following substitution pattern, which is repeated from nucleotide eleven, counting from the 3' end, to the 5' end: XXXxXXXx, XXxXxXxX, XXXxxxXX or XXxXxxXX, wherein "X" denotes an LNA unit and "x" denotes a non-LNA unit.

The specific substitution pattern for the nucleotides from position 11, counting from the 3' end, to the 5' end depends on the number of nucleotides in the mixmer. In a preferred embodiment, the mixmer contains 12 nucleotides and the substitution pattern for positions 11 to 12, counting from the 3' end, is selected from the group consisting of xX and Xx, wherein "X" denotes an LNA unit and "x" denotes a non-LNA unit. In some embodiments, the substitution pattern for positions 11 to 12, counting from the 3' end, is xX, wherein "X" denotes an LNA unit and "x" denotes a non-LNA unit. Alternatively, no LNA units are present in positions 11 to 12, counting from the 3' end, i.e. the substitution pattern is xx.

In some embodiments, the mixmer contains 12 nucleotides and the substitution pattern for positions 10 to 12, counting from the 3' end, is selected from the group consisting of Xxx, xXx, xxX, XXx, XxX, xXX and XXX, wherein "X" denotes an LNA unit and "x" denotes a non-LNA unit. In some embodiments thereof, the substitution pattern for positions 10 to 12, counting from the 3' end, is selected from the group consisting of xXx, xxX and xXX, wherein "X" denotes an LNA unit and "x" denotes a non-LNA unit. In some embodiments, the substitution pattern for positions 10 to 12, counting from the 3' end, is xxX, wherein "X" denotes an LNA unit and "x" denotes a non-LNA unit. Alternatively, no LNA units are present in positions 10 to 12, counting from the 3' end, *i.e.* the substitution pattern is xxx.

In some embodiments, the mixmer contains an LNA unit at the 5' end. In some embodiments, the mixmer contains an LNA unit at the first two positions, counting from the 5' end. The mixmer may also contain one or more of the structural features which are specified in the context of the antimiR herein - either the context that the mixmer contains a similar pattern and number of nucleotides/nucleotide analogues (e.g. X and x or X and Y). In some embodiments where the contiguous nucleotide sequence of the mixmer (as a second oligomer) is complementary to the contiguous nucleotide sequence of antimiR (the first oligomer) or sub-sequence thereof, the corresponding pattern of nucleotide analogues may be such so that one or more, such as 2, 3, 4, 5, 6, 7, 8, 9, 10, 11, 12 of even all the nucleotide analogues present in the second oligomer form hydrogen bonds with complementary nucleotide analogues in the first oligomer. As is discussed herein, this is desirable as it results in a very stable duplex between the two oligomers which effectively results in deactivation of the first oligomer (and therefore reduction in bioavailability).

As is described herein, the design of the mixmer antidote (second oligmers), is preferably coordinated with the position of nucleotide analogues in the first oligomer. In some embodiments the first oligomer comprises one or more 2' substituted nucleotide analogues such as a nucleotide analogue selected from the group consisting of, 2'-O-alkyl-RNA unit, 2'-OMe-RNA unit, 2'-amino-DNA unit, 2'-fluoro-DNA unit, 2'-MOE-RNA unit - the second oligomer may therefore comprise a similar 2' substituted nucleotide analogue or, preferably LNA, at the corresponding position in the second oligomer. When the first oligomer is a mixmer, the second oligomer may also be a mixmer which has nucloeitde analogues (such as LNA) at the corresponding positions to the nucleotide analgues in the first oligomer. When the first oligomer is a gapmer - the second oligmer may be a mixmer which comprises at least one nucleotide analogue, such as 2 or 3 nucleotide analogues, in a position (or positions) which corresponds to a nucleotide analogue(s) in region A or C of the gapmer.

### Tailmers and Headmers

A headmer is defined by a contiguous stretch of nucleotide analogues at the 5'-end followed by a contiguous stretch of DNA (or modified nucleotides units recognizable and cleavable by the RNase, such as RNAseH) towards the 3'-end (such as at least 6 or at least 7 of such nucleotides), and a tailmer is defined by a contiguous stretch of DNA (or modified monomers recognizable and cleavable by the RNase, such asRNaseH), at the 5'-end (such as at least 6 or at least 7 such nucleotides), followed by a contiguous stretch of nucleotide analogues towards the 3'-end.

### Totalmers

A totalmer is a single stranded oligomer which only comprises non-naturally occurring nucleotides.

The first and/or second oligomer according to the invention maybe totalmers - indeed various totalmer designs are highly effective as first oligomers, particularly when targeting microRNA (antimiRs) or as splice switching oligomers (SSOs). The second oligomer may also be a totalmer and indeed, due to their ability to effectively and specifically bind to their target, the use of totalmers as second oligomers are considered to be particularly effective in decreasing the bioavailability of the first oligomer.

In some embodiments, the totalmer comprises or consists of at least one XYX or YXY sequence motif, such as a repeated sequence XYX or YXY, wherein X is LNA and Y is an alternative (*i.e.* non LNA) nucleotide analogue, such as a 2'-OMe RNA unit and 2'-fluoro DNA unit. The above sequence motif may, in some embodiments, be XXY, XYX, YXY or YYX for example.

In some embodiments, the totalmer may comprise or consist of a contiguous nucleotide sequence of between 8 and 16 nucleotides, such as 9, 10, 11, 12, 13, 14, or 15 nucleotides, such as between 8 and 12 nucleotides.

In some embodiments, the contiguous nucleotide sequence of the totolmer comprises of at least 30%, such as at least 40%, such as at least 50%, such as at least 60%, such as at least 70%, such as at least 80%, such as at least 90%, such as 95%, such as 100% LNA units. The remaining units may be selected from the non-LNA nucleotide analgues referred to herein in, such those selected from the group consisting of 2'-O_alkyl-RNA unit, 2'-OMe-RNA unit, 2'-amino-DNA unit, 2'-fluoro-DNA unit, LNA unit, PNA unit, HNA unit, INA unit, and a 2'MOE RNA unit, or the group 2'-OMe RNA unit and 2'-fluoro DNA unit.

In some embodiments the totalmer consist or comprises of a contiguous nucleotide sequence which consists only of LNA units.

In some embodiments, the totalmer (as the first oligomer) may be targeted against a microRNA (*i.e.* be antimiRs) - as referred to in US provisional applications 60/979217 and 61/028062, and WO 2009/043353.

### MicroRNA modulation via the first oligomer.

In some embodiments, the first oligomer is an antimiR, which comprises or consists of a contiguous nucleotide sequence which is corresponds to or is fully complementary to the entire mature microRNA. The use of the present invention in controlling the *in vivo* activity of microRNA is considered of primary importance due to the fact that microRNAs typically regulate numerous mRNAs in the subject. The ability to inactivate therapeutic antimiRs is therefore very desirable.

Numerous microRNAs are related to a number of diseases. For example:
Examples of therapeutic indications which may be treated by the pharmaceutical compositions of the invention:

| **microRNA** | **Possible medical indications** |
|---|---|
| miR-1 | Cardiac arythmia |
| miR-21 | Glioblastoma, breast cancer, hepatocellular carcinoma, colorectal cancer, sensitization of gliomas to cytotoxic drugs, cardiac hypertrophy |
| miR-21, miR-200b and miR-141 | Response to chemotherapy and regulation of cholangiocarcinoma growth |
| miR-122 | hypercholesterolemia, hepatitis C infection, hemochromatosis |
| miR-19b | lymphoma and other tumour types |
| miR-26a | Osteoblast differentiation of human stem cells |
| miR-155 | lymphoma, pancreatic tumor development, breast and lung cancer |
| miR-203 | Psoriasis |
| miR-375 | diabetes, metabolic disorders, glucose-induced insulin secretion from pancreatic endocrine cells |
| miR-181 | myoblast differentiation, auto immune disorders |
| miR-10b | Breast cancer cell invasion and metastasis |
| miR-125b-1 | Breast, lung, ovarian and cervical cancer |
| miR-221 and 222 | Prostate carcinoma, human thyroid papillary car, human hepatocellular carcinoma |
| miRNA-372 and - 373 | testicular germ cell tumors. |
| miR-142 | B-cell leukemia |
| miR-17 - 19b cluster | B-cell lymphomas, lung cancer, hepatocellular carcinoma |

Tumor suppressor gene tropomysin 1 (TPM1) mRNA has been indicated as a target of miR-21. Myotrophin (mtpn) mRNA has been indicated as a target of miR 375.
The first oligomer may therefore be an antimir which targets (i.e. comprises or consists of a contiguous nucleotide sequence which is fully complementary to (a corresponding region of) one of the microRNAs listed above or comprises of no more than a single mismatch thereto.

Hence, some aspects of the invention relates to the treatment of a disease associated with the expression of microRNAs selected from the group consisting of infectious diseases such as viral diseases such as hepatitis C virus and HIV, fragile X mental retardation, inflammatory diseases, cancer, such as chronic lymphocytic leukemia, breast cancer, lung cancer and colon cancer.

MicroRNAs (miRNAs) are an abundant class of short endogenous RNAs that act as post-transcriptional regulators of gene expression by base-pairing with their target mRNAs. The mature miRNAs are processed sequentially from longer hairpin transcripts by the RNAse III ribonucleases Drosha. Mature microRNAs (miRs) typically between 20 and 25 contiguous RNA nucleotides. It is now widely established that several microRNAs are associated with medical conditions and disease, and several companies are developing therapeutics based on oligomers which either mimic microRNAs or specifically hybridse to specific microRNAs associated with disease phenotypes - such oligomers are referred to, herein, as microRNA mimics and antimiRs respectfully, and the first oligomer, in some embodiments may be such microRNA modulating oligomers.

In some embodiments the first oligomer according to the invention, consists or comprises of a contiguous nucleotide sequence which corresponds to or is fully complementary to a microRNA sequence, such as a mature microRNA sequence, such as the human microRNAs published in miRBase (http://microrna.sanger.ac.uk/cgi-bin/sequences/mirna_summary.pl?org=hsa). In some embodiment the microRNA is a viral microRNA. At the time of writing, there are 695 human miRNA sequences in miRBase, including the mature microRNA sequence of each human microRNA. Other human microRNAs which may be targeted by the first oligomer include those disclosed in WO08040355A. In some embodiments the first oligomer according to the invention, consists or comprises of a contiguous nucleotide sequence which corresponds to or is fully complementary to a microRNA sequence selected from the group consisting of hsa-miR19b, hsa-miR21, hsa-miR 122, hsa-miR 142 a7b, hsa-miR 155, and hsa-miR 375. In some embodiments the first oligomer according to the invention, consists or comprises of a contiguous nucleotide sequence which corresponds to or is fully complementary to a microRNA sequence selected from the group consisting of hsa-miR221 and hsa-miR222. In some embodiments the first oligomer according to the invention, consists or comprises of a contiguous nucleotide sequence which corresponds to or is fully complementary to hsa-miR122.

### AntimiR oligomers

Preferred first oligomer 'antimiR' designs and oligomers are disclosed in WO2007/112754, WO2007/112753, WO 2009/043353 and US provisional applications 60/979217 and 61/028062. In some embodiments, the first oligomer is an antimiR which is a mixmer or a totalmer.

AntimiR oligomers are oligomers which consist or comprise of a contiguous nucleotide sequence which is fully complementary to, or essentially complementary to (*i.e.* may comprise one or two mismatches), to a microRNA sequence, or a corresponding sub-sequence thereof. In this regards it is considered that the antimiR may be comprise a contiguous nucleotide sequence which is complementary or essentially complementary to the entire mature microRNA, or the antimiR may be comprise a contiguous nucleotide sequence which is complementary or essentially complementary to a sub-sequence of the mature microRNA or pre-microRNA - such a sub-sequence (and therefore the corresponding contiguous nucleotide sequence) is typically at least 8 nucleotides in length, such as between 8 and 25 nucleotides, such as 9, 10, 11, 12, 13, 14, 15, 16, 17, 18, 19, 20, 21, 22, 23, 24 nucleotides in length, such as between 10-17 or 10-16 nucleotides, such as between 12 - 15 nucleotides.

Numerous designs of AnitmiRs have been suggested, and typically antimiRs for therapeutic use, such as the contiguous nucleotide sequence thereof comprise one or more nucleotide analogues units.

In some embodiments the antimiR may have a gapmer structure as herein described. However, as explained in WO2007/112754 and WO2007/112753, other designs may be preferable, such as mixmers, or totalmers.

WO2007/112754 and WO2007/112753, provide antimiR oligomers and antimiR oligomer designs where the oligomers which are complementary to mature microRNA

In some embodiments, a subsequence of the antimiR corresponds to the miRNA seed region. In some embodiments, the first or second 3' nucleobase of the oligomer corresponds to the second 5' nucleotide of the microRNA sequence.

In some antimiR embodiments, nucleobase units 1 to 6 (inclusive) of the oligomer as measured from the 3' end the region of the oligomer are complementary to the microRNA seed region sequence.

In some antimiR embodiments, nucleobase units 1 to 7 (inclusive) of the oligomer as measured from the 3' end the region of the oligomer are complementary to the microRNA seed region sequence.

In some e antimiR mbodiments, nucleobase units 2 to 7 (inclusive) of the oligomer as measured from the 3' end the region of the oligomer are complementary to the microRNA seed region sequence.

In some embodiments, the antimiR oligomer comprises at least one nucleotide analogue unit, such as at least one LNA unit, in a position which is within the region complementary to the miRNA seed region. The antimiR oligomer may, in some embodiments comprise at between one and 6 or between 1 and 7 nucleotide analogue units, such as between 1 and 6 and 1 and 7 LNA units, in a position which is within the region complementary to the miRNA seed region.

In some embodiments, the antimiR of the invention is 7, 8 or 9 nucleotides long, and comprises a contiguous nucleotide sequence which is complementary to a seed region of a human or viral microRNA, and wherein at least 80 %, such as 85%, such as 90%, such as 95%, such as 100% of the nucleotides are LNA.

In some embodiments, the antimiR of the invention is 7, 8 or 9 nucleotides long, and comprises a contiguous nucleotide sequence which is complementary to a seed region of a human or viral microRNA, and wherein at least 80 % of the nucleotides are LNA, and wherein at least 80%, such as 85%, such as 90%, such as 95%, such as 100% of the internucleotide bonds are phosphorothioate bonds.

In some embodiments, the antimiR comprises one or two LNA units in positions three to eight, counting from the 3' end. This is considered advantageous for the stability of the A-helix formed by the oligo:microRNA duplex, a duplex resembling an RNA:RNA duplex in structure.

The table on pages 48 line 15 to page 51, line 9 of WO2007/112754 provides examples of anti microRNA oligomers (*i.e.* antimiRs which may be the first oligomer).

### MicroRNA mimics

In some embodiments the first oligomer is in the form of a miRNA mimic which can be introduced into a cell to repress the expression of one or more mRNA target(s). miRNA mimics are typically fully complementary to the full length miRNA sequence. miRNA mimics are compounds comprising a contiguous nucleotide sequence which are homologous to a corresponding region of one, or more, of the miRNA sequences provided or referenced to herein. The use of miRNA mimics or antimiRs can be used to (optionally) further repress the mRNA targets, or to silence (down- regulate) the miRNA, thereby inhibiting the function of the endogenous miRNA, causing derepression and increased expression of the mRNA target. The invention therefore provides for a method for the derepression of a mRNA target in a subject, said method comprising the step of administering a second oligomer according to the invention, wherein the mRNA target is repressed by the presence of a microRNA mimic (which has been administered prior to the administration of the second oligomer), where in the second oligomer conmprises or consists of a contiguous nucleotide sequence which is complementary to, or essentially complementary to, a contiguous nucleotide sequence of the miRNA mimic or sub-sequence thereof (*i.e.* corresponds to said miRNA mimic contiguous nucleotide sequence.

### Aptamers

In some embodiments the first oligomer may be an aptamer, a spiegelmer. Aptamers (also referred to as *Spiegelmers)* in the context of the present invention as nucleic acids of between 20 and 50 nucleotides in length, which have been selected on the basis of their conformational structure rather than the sequence of nucleotides - they elicit their therapeutic effect by binding with a target protein directly *in vivo* and they do not, therefore, comprise of the reverse complement of their target - indeed their target is not a nucleic acid but a protein. Specific aptamers which may be the first oligomer include Macugen (OSI Pharmaceuticals) or ARC1779, (Archemix, Cambridge, MA). In some embodiments, the first oligomer is not an aptamer. In some embodiments the first oligomer is not an aptamer or a spiegelmer.

### siRNA Complexes

In some embodiments, the first oligomer may be part of a siRNA complex - *i.e.* the antisense or passenger strand of the siRNA complex.

In some embodiments the siRNA complex comprises two single stranded oligomers of between 17 - 25 nts in length, such as 18, 19, 20, 21, 22, 23, 24 nucleotides in length, such as between 21-23 nucleotides in length. In some embodiments, the sense and/or antisense strand of the siRNA may comprise a 3' overhang, typically of 1, 2 or 3 nucleotides. Suitably, the sense and or antisense strand may comprise one or more nucleotide analogues.

In some embodiments the siRNA complex is a siLNA, such as the siRNA designs described in WO2004/000192, WO2005/073378, WO2007/085485.

In some embodiments, the siRNA complex is a sisiLNA, such as those described in WO2007/107162.

### First and Second Oligomers

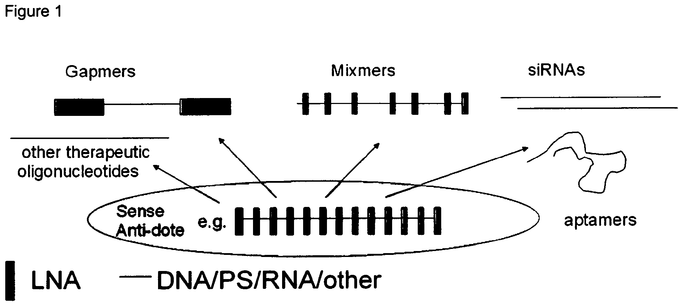

The principle of the invention is illustrated in figure 1 which shows how the antidote oligomer, which is in this case represented by a mixmer oligomer, but may also be one of the other second oligomer designs referred to here, can target a range of therapeutic oligomers (first oligomers). The second oligomer may block the activity of the first oligomer stoichiometrically and in a sequence dependant and highly specific manner. In a preferred aspect, this is facilitated by the use of LNA nucleotide analogues in the second oligomer, optionally in conjunction with other nucleotide analogues, such as 2'MOE or 2' fluoro, as described above.

The first oligomer may also comprise nucleotide analogues, such as LNA and/or 2' substituted nucleotide analogues. Indeed it is considered that, in some embodiments, that the second oligomer may be designed so that the duplex between the first oligomer and the complementary second oligomer results in the formation of nucleotide analogue base pairs - *i.e.* as least one nucleotide analogue, such as LNA or 2' substituted nucleotide analogues, of the second oligomer is positioned so that it is in a position which corresponds to a nucleotide analogue in the first oligomer - the formation of the duplex therefore results in a hydrogen bond between the corresponding bases of the two nucleotide analogues. In some embodiments, the second oligomer comprises of a nucleotide analogue, such as LNA or 2' substituted nucleotide analogues, at each position which corresponds to (*i.e.* is complementary to) a nucleotide analogue in the first oligomer. In some embodiments, the second oligomer comprises of a nucleotide analogue at a number of positions which corresponds to (*i.e.* is complementary to) a nucleotide analogue in the first oligomer, for example, 2, 3, 4, 5, 6, 7, 8, 9, 10, 11, or 12 complementary positions. It is, in some preferred aspects that at least one of the corresponding nucleotide analogues which form base pairs upon duplex formation between the first and second oligomers are LNA, and in some embodiments, that both the corresponding nucleotide analogues are LNA. Indeed, due to the very high stability of LNA:LNA duplexes, the formation of such duplexes is considered to be particularly effective in reducing the bioavailability of the first oligomer.

In some embodiments however, the duplex formation between one or more corresponding nucleotide analogues present in the first and second oligomers which form base pairs upon duplex formation between the first and second oligomers are LNA/2'MOE or LNA/2'fluoro or 2'MOE/LNA or 2'fluoro/LNA (first oligmer/second oligomer).

In some embodiments the first oligomer is a gapmer and the second oligomer is a mixmer or a totalmer. In some embodiments the first oligomer is a mixmer and the second oligomer is a mixmer or a totalmer according to the invention. In some embodiments the first oligomer is a totalmer and the second oligomer is a mixmer or a totalmer according to the invention. In some embodiments the first oligomer is a antimiR and the second oligomer is a mixmer or a totalmer according to the invention. In some embodiments the first oligomer is a antisense oligomer and the second oligomer is a mixmer or a totalmer according to the invention. In some embodiments the first oligomer is the sense or antisense strand of a siRNA or siLNA complex and the second oligomer is a mixmer or a totalmer according to the invention.

### The Second Oligomer

Suitably the second oligomer of the invention is capable of down-regulating the bioavailability of the first oligomer. In this regards, the second oligomer of the invention reduces the bioavailability of the first oligomer typically in a mammalian such as a human cell. The reduction on bioavailability can be measured by monitoring the target of the first oligomer, or the phenotypic effect of the first oligomer, or in some embodiments, the accumulation of the first oligomer/second oligomer complexes. In some embodiments, the second oligomers of the invention bind to or are capable of binding to the first oligomer to effect a in vivo reduction of 'bioavailable' first oligomer of at least 10% or 20% compared to the level of the first oligomer during standard therapeutic treatment, more preferably at least a 30%, 40%, 50%, 60%, 70%, 80%, 90% or 95%.. In the same or different embodiments, the reduction on bioavailability is less than 100%, such as less than 98% inhibition, less than 95% inhibition, less than 90% inhibition, less than 80% inhibition, such as less than 70% inhibition. Modulation of expression level may be determined by measuring the first oligomer target protein levels, e.g. by the methods such as SDS-PAGE followed by western blotting using suitable antibodies raised against the target protein. Alternatively, modulation of expression levels can be determined by measuring levels of the first oligomer nucleic acid or its target (e.g. microRNA - or mRNA) *e.g*. by northern blotting or quantitative RT-PCR. In some preferred embodiment, the second oligomer may be in the form of a mixmer or a totalmer according to the invention.

When designing the second oligomer it is desirable to BLAST the proposed sequence to minimise the risk that the second oligomer will hybridise against off-target sequences, such as RNA sequences present in the genome - such as the human genome.

The second oligomer is suitably an oligomer of length between 6 and 30 nucleotides in length, which comprises or consists of a contiguous nucleotide sequence, which typically is of at least 6, or at least 7, or at least 8, or at least 9 nucleotides in length. The contiguous nucleotide sequence of the second oligomer may be between 8 and 30 nucleotides in length. In some embodiments the length of the second oligomer, or the contiguous nucleotide sequence thereof is between 10 and 30 nucleotides, such as between 12 and 25 nucleotides, such as 13, 14, 15, 16, 17, 18, 19, 20 ,21, 22, 23 or 24 nucleotides in length, or between 10 and 18 nucleotides, or between 10 and 16 nucleotides, such as 10, 11, 12, 13, 14, 15 and 16 nucleotides, or between 12 and 16, or between 12 and 14 nucleotides.

In some embodiments, the second oligomer consists of the contiguous nucleotide sequence. In some embodiments all the nucleotides present in the second oligomer are within the contiguous nucleotide sequence - the oligomer may be conjugated as referred to herein.

The invention provides a method of down-regulating or inhibiting the effect of a first oligomer in a cell which is expressing, said method comprising administering the second oligomer to said cell to down-regulating or inhibiting the first oligomer. Suitably the cell is a mammalian cell such as a human cell, and suitably is in a subject. The administration may occur, in some embodiments, *in vitro.* The administration may occur, some embodiments, *in vivo.*

In some embodiments, the second oligomer is used in a tissue specific manner - for example to selectively reduce the bioavailability of the first oligomer in certain tissues - for example the liver (e.g. via use of the GaINAc system). As described below, the biodistribution of the first and or second oligomers of the invention may also be regulated, in the case of phosphorothioate oligomers, by the insertion of one or two phosphodiester linkages into the respective oligomer, such as adjacent to or in between nucleotide analogue units.

In some embodiments, it is considered beneficial to reduce the bioavailability of the first oligomer (*i.e.* activity of the first oligomer) in specific host organ or tisses, whilst retaining bioavailability in other tissues - for example it may be beneficial to reduce the bioavailability in the liver whist maintaining the bioavailability in a tumor tissue - thereby increasing the therapeutic window.

In some embodiments, the second oligomers of the invention, the 5' and 3' nucleotides are nucleotide analogues, such as LNA nucleotides. In some embodiments, the second oligomer does not comprise more than 2 or more than 3 consecutive naturally occurring nucleotides such as DNA nucleotides.

In some embodiments, the second oligomers of the invention, the internucleoside linkages may be phosphorothioate, although due to the high load of LNA nucleotides the internucleoside linkages may, in some embodiments also be phosphodiester, or a combination of phosphorothioate and phosphodiester - for example, in some embodiments, one or more of the internucleotides between two LNA nucleotides, or adjacent to an LNA nucleotide may be phosphodiester linkages, where as the remaining internucleotides are phosphorothioate.

### Nucleotide analogues

In some embodiments, the terms "nucleoside", "nucleotide", "nucleobase", "base", "unit" and "monomer" are used interchangeably. In some embodiments, the terms "nucleoside analogue" and "nucleotide analogue" are used interchangeably.

The term "nucleotide" as used herein, refers to a glycoside comprising a sugar moiety, a base moiety and a covalently linked group (linkage group) such as a phosphate or phosphorothioate internucleotide linkage group, and covers both naturally occurring nucleotides, such as DNA or RNA, and non-naturally occurring nucleotides comprising modified sugar and/or base moieties, which are also referred to as "nucleotide analogues" herein. Herein, a single nucleotide (unit) may also be referred to as a monomer or nucleic acid unit.

In field of biochemistry, the term "nucleoside" is commonly used to refer to a glycoside comprising a sugar moiety and a base moiety, and may therefore be used when referring to the nucleotide units, which are covalently linked by the internucleotide linkages between the nucleotides of the oligomer.

The person having ordinary skill in the art would understand that, in the context of the present invention, the 5' terminal nucleotide of an oligonucleotide (oligomer) does not comprise a 5' internucleotide linkage group, although it may or may not comprise a 5' terminal group.

Non-naturally occurring nucleotides include nucleotides which have modified sugar moieties, such as bicyclic nucleotides or 2' modified nucleotides, such as 2' substituted nucleotides.

The terms "corresponding nucleotide analogue" and "corresponding nucleotide" are intended to indicate that the nucleotide in the nucleotide analogue and the naturally occurring nucleotide are identical. For example, when the 2-deoxyribose unit of the nucleotide is linked to an adenine, the "corresponding nucleotide analogue" contains a pentose unit (different from 2-deoxyribose) linked to an adenine.

"Nucleotide analogues" are variants of natural nucleotides, such as DNA or RNA nucleotides, by virtue of modifications in the sugar and/or base moieties. Analogues could in principle be merely "silent" or "equivalent" to the natural nucleotides in the context of the oligomer, *i.e.* have no functional effect on the way the oligomer works to inhibit target gene expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell. Specific examples of nucleoside analogues are described by *e.g.* Freier & Altmann; Nucl. Acid Res., 1997, 25, 4429-4443 and Uhlmann; Curr. Opinion in Drug Development, 2000, 3(2), 293-213, and in Scheme 1: 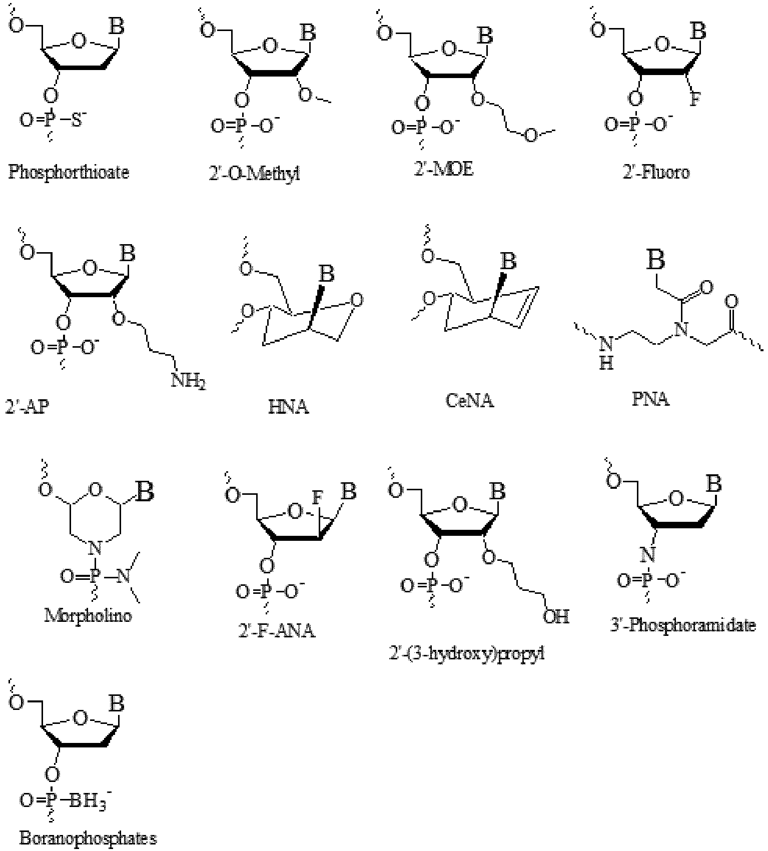 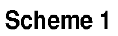 The oligomer may thus comprise or consist of a simple sequence of natural occurring nucleotides - preferably 2'-deoxynucleotides (referred to here generally as "DNA"), but also possibly ribonucleotides (referred to here generally as "RNA"), or a combination of such naturally occurring nucleotides and one or more non-naturally occurring nucleotides, *i.e.* nucleotide analogues. Such nucleotide analogues may suitably enhance the affinity of the oligomer for the target sequence.

Examples of suitable and preferred nucleotide analogues are provided by WO2007/031091 or are referenced therein.

Incorporation of affinity-enhancing nucleotide analogues in the oligomer, such as LNA or 2'-substituted sugars, can allow the size of the specifically binding oligomer to be reduced, and may also reduce the upper limit to the size of the oligomer before non-specific or aberrant binding takes place.

In some embodiments, the oligomer or oligomers comprises at least 1 nucleoside analogue. In some embodiments the oligomer or oligomers comprise at least 2 nucleotide analogues. In some embodiments, the oligomer or oligomers comprises from 3-8 nucleotide analogues, *e.g*. 6 or 7 nucleotide analogues. In the by far most preferred embodiments, particularly in relation to the second oligomer, but may also refer to the first oligomer, at least one of said nucleotide analogues is a locked nucleic acid (LNA); for example at least 3 or at least 4, or at least 5, or at least 6, or at least 7, or 8, of the nucleotide analogues may be LNA. In some embodiments all the nucleotides analogues may be LNA.

Examples of such modification of the nucleotide include modifying the sugar moiety to provide a 2'-substituent group or to produce a bridged (locked nucleic acid) structure which enhances binding affinity and may also provide increased nuclease resistance.

A preferred nucleotide analogue is LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA). Most preferred is beta-D-oxy-LNA.

In some embodiments the nucleotide analogues present within the oligomer or oligomers are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid -Christensen, 2002. Nucl. Acids. Res. 2002 30: 4918-4925) units and 2'MOE units. In some embodiments there is only one of the above types of nucleotide analogues present in the oligomer of the invention, or contiguous nucleotide sequence thereof.

In some embodiments the nucleotide analogues are 2'-O-methoxyethyl-RNA (2'MOE), 2'-fluoro-DNA monomers or LNA nucleotide analogues, and as the oligomer or oligomers may comprise nucleotide analogues which are independently selected from these three types of analogue, or may comprise only one type of analogue selected from the three types. In some embodiments at least one of said nucleotide analogues is 2'-MOE-RNA, such as 2, 3, 4, 5, 6, 7, 8, 9 or 10 2'-MOE-RNA nucleotide units. In some embodiments at least one of said nucleotide analogues is 2'-fluoro DNA, such as 2, 3, 4, 5, 6, 7, 8, 9 or 10 2'-fluoro-DNA nucleotide units.

In some embodiments, the second oligomer comprises both LNA and 2'-MOE-RNA or 2'-fluoro nucleotides, and may, in some embodiment consist of LNA and 2'-MOE, or LNA and 2'-fluoro nucleotides.

In some embodiments, the oligomer or oligomers comprises at least one Locked Nucleic Acid (LNA) unit, such as 1, 2, 3, 4, 5, 6, 7, or 8 LNA units, such as between 3 - 7 or 4 to 8 LNA units, or 3, 4, 5, 6 or 7 LNA units. In some embodiments, all the nucleotide analogues are LNA. In some embodiments, the oligomer may comprise both beta-D-oxy-LNA, and one or more of the following LNA units: thio-LNA, amino-LNA, oxy-LNA, and/or ENA in either the beta-D or alpha-L configurations or combinations thereof. In some embodiments all LNA cytosine units are 5'methyl-Cytosine. In some embodiments of the invention, the oligomer or oligomers, may comprise both LNA and DNA units. Preferably the combined total of LNA and DNA units is 10-25, preferably 10-20, even more preferably 12-16. In some embodiments, the nucleotide sequence of the oligomer, such as the contiguous nucleotide sequence consists of at least one LNA and the remaining nucleotide units are DNA units. In some embodiments, the oligomer or oligomers, comprises only LNA nucleotide analogues and naturally occurring nucleotides (such as RNA or DNA, most preferably DNA nucleotides), optionally with modified internucleotide linkages such as phosphorothioate.

The term "nucleobase" refers to the base moiety of a nucleotide and covers both naturally occuring a well as non-naturally occurring variants. Thus, "nucleobase" covers not only the known purine and pyrimidine heterocycles but also heterocyclic analogues and tautomeres thereof.

Examples of nucleobases include, but are not limited to adenine, guanine, cytosine, thymidine, uracil, xanthine, hypoxanthine, 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

In some embodiments, at least one of the nucleobases present in the oligomer or oligomers is a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### LNA

The term "LNA" refers to a bicyclic nucleotide analogue, known as "Locked Nucleic Acid". It may refer to an LNA monomer, or, when used in the context of an "LNA oligonucleotide", LNA refers to an oligonucleotide containing one or more such bicyclic nucleotide analogues. LNA nucleotides are characterised by the presence of a biradical 'bridge' between C2' and C4' of the ribose sugar ring -for example as shown as the biradical R^{4*} - R^{2*} as described below.

The LNA used in the oligonucleotide compounds of the invention preferably has the structure of the general formula I 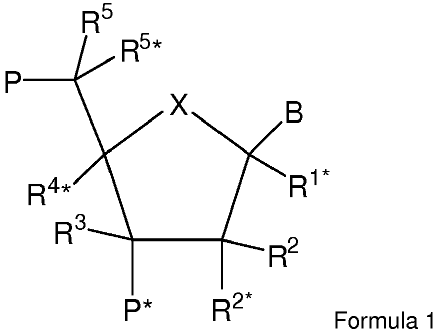
wherein for all chiral centers, asymmetric groups may be found in either R or S orientation;
wherein X is selected from -O-, -S-, -N(R^{N*})-, -C(R⁶R^{6*})-, such as, in some embodiments -O-;
B is selected from hydrogen, optionally substituted C₁₋₄-alkoxy, optionally substituted C₁₋₄-alkyl, optionally substituted C₁₋₄-acyloxy, nucleobases including naturally occurring and nucleobase analogues, DNA intercalators, photochemically active groups, thermochemically active groups, chelating groups, reporter groups, and ligands, preferably a nucleobase;
P designates an internucleotide linkage to an adjacent monomer, or a 5'-terminal group, such internucleotide linkage or 5'-terminal group optionally including the substituent R⁵ or equally applicable the substituent R^{5*};
P* designates an internucleotide linkage to an adjacent monomer, or a 3'-terminal group;
R^{4*} and R^{2*} together designate a biradical consisting of 1 - 4 groups/atoms selected from -C(R^{a}R^{b})-, -C(R^{a})=C(R^{b})-, -C(R^{a})=N-, -O-, -Si(R^{a})₂-, -S-, -SO₂-, -N(R^{a})-, and >C=Z, wherein Z is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, optionally substituted C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂), wherein for all chiral centers, asymmetric groups may be found in either *R* or *S* orientation, and;
each of the substituents R^{1*}, R², R³, R⁵, R^{5*}, R⁶ and R^{6*}, which are present is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene; ; wherein R^{N} is selected from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof. For all chiral centers, asymmetric groups may be found in either *R* or *S* orientation.

In some embodiments, R^{4*} and R^{2*} together designate a biradical consisting of a groups selected from the group consisting of C(R^{a}R^{b})-C(R^{a}_{R}^{b})-, C(R^{a}R^{b})-O-, C(R^{a}R^{b})-NR^{a}-, C(R^{a}R^{b})-S-, and C(R^{a}R^{b})-C(R^{a}R^{b})-O-, wherein each R^{a} and R^{b} may optionally be independently selected. In some embodiments, R^{a} and R^{b} may be, optionally independently selected from the group consisting of hydrogen and C₁₋₆alkyl, such as methyl, such as hydrogen.

In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. For all chiral centers, asymmetric groups may be found in either *R* or *S* orientation.

In some embodiments, R^{1*}, R², R³, R⁵, R^{5*} are hydrogen.

In some embodiments, R^{1*}, R², R³ are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. For all chiral centers, asymmetric groups may be found in either *R* or *S* orientation.

In some embodiments, R^{1*}, R², R³ are hydrogen.

In some embodiments, R⁵ and R^{5*} are each independently selected from the group consisting of H, -CH₃, -CH₂-CH₃,- CH₂-O-CH₃, and -CH=CH₂. Suitably in some embodiments, either R⁵ or R^{5*} are hydrogen, where as the other group (R⁵ or R^{5*} respectively) is selected from the group consisting of C₁₋₅ alkyl, C₂₋₆ alkenyl, C₂₋₆ alkynyl, substituted C₁₋₆ alkyl, substituted C₂₋₆ alkenyl, substituted C₂₋₆ alkynyl or substituted acyl (-C(=O)-); wherein each substituted group is mono or poly substituted with substituent groups independently selected from halogen, C₁₋₆ alkyl, substituted C₁₋₆ alkyl, C₂₋₆ alkenyl, substituted C₂₋₆ alkenyl, C₂₋₆ alkynyl, substituted C₂₋₆ alkynyl, OJ₁, SJ₁, NJ₁J₂, N₃, COOJ₁, CN, O-C(=O)NJ₁J₂, N(H)C(=NH)NJ,J₂ or N(H)C(=X)N(H)J₂ wherein X is O or S; and each J₁ and J₂ is, independently, H, C₁₋₆ alkyl, substituted C₁₋₆ alkyl, C₂₋₆ alkenyl, substituted C₂₋₆ alkenyl, C₂₋₆ alkynyl, substituted C₂₋₆ alkynyl, C₁₋₆ aminoalkyl, substituted C₁₋₆ aminoalkyl or a protecting group. In some embodiments either R⁵ or R^{5*} is substituted C₁₋₆ alkyl. In some embodiments either R⁵ or R^{5*} is substituted methylene wherein preferred substituent groups include one or more groups independently selected from F, NJ₁J₂, N₃, CN, OJ₁, SJ₁, O-C(=O)NJ₁J₂, N(H)C(=NH)NJ, J₂ or N(H)C(O)N(H)J₂. In some embodiments each J₁ and J₂ is, independently H or C₁₋₆ alkyl. In some embodiments either R⁵ or R^{5*} is methyl, ethyl or methoxymethyl. In some embodiments either R⁵ or R^{5*} is methyl. In a further embodiment either R⁵ or R^{5*} is ethylenyl. In some embodiments either R⁵ or R^{5*} is substituted acyl. In some embodiments either R⁵ or R^{5*} is C(=O)NJ₁J₂. For all chiral centers, asymmetric groups may be found in either *R* or *S* orientation. Such 5' modified bicyclic nucleotides are disclosed in WO 2007/134181.

In some embodiments B is a nucleobase, including nucleobase analogues and naturally occurring nucleobases, such as a purine or pyrimidine, or a substituted purine or substituted pyrimidine, such as a nucleobase referred to herein, such as a nucleobase selected from the group consisting of adenine, cytosine, thymine, adenine, uracil, and/or a modified or substituted nucleobase, such as 5-thiazolo-uracil, 2-thio-uracil, 5-propynyl-uracil, 2'thio-thymine, 5-methyl cytosine, 5-thiozolo-cytosine, 5-propynyl-cytosine, and 2,6-diaminopurine.

In some embodiments, R^{4*} and R^{2*} together designate a biradical selected from-C(R^{a}R^{b})-O-, -C(R^{a}R^{b})-C(R^{c}R^{d})-O-, -C(R^{a}R^{b})-C(R^{c}R^{d})-C(R^{e}R^{f})-O-, -C(R^{a}R^{b})-O-C(R^{c}R^{d})-,-C(R^{a}R^{b})-O-C(R^{c}R^{d})-O-, -C(R^{a}R^{b})-C(R^{c}R^{d})-, -C(R^{a}R^{b})-C(R^{c}R^{d})-C(R^{e}R^{f})-,-C(R^{a})=C(R^{b})-C(R^{c}R^{d})-, -C(R^{a}R^{b})-N(R^{c})-, -C(R^{a}R^{b})-C(R^{c}R^{d})-N(R^{e})-, -C(R^{a}R^{b})-N(R^{c})-O-, and-C(R^{a}R^{b})-S-, -C(R^{a}R^{b})-C(R^{c}R^{d})-S-, wherein R^{a}, R^{b}, R^{c}, R^{d}, R^{e}, and R^{f} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂). For all chiral centers, asymmetric groups may be found in either *R* or *S* orientation.

In a further embodiment R^{4*} and R^{2*} together designate a biradical (bivalent group) selected from -CH₂-O-, -CH₂-S-, -CH₂-NH-, -CH₂-N(CH₃)-, -CH₂-CH₂-O-, -CH₂-CH(CH₃)-,-CH₂-CH₂-S-, -CH₂-CH₂-NH-, -CH₂-CH₂-CH₂-, -CH₂-CH₂-CH₂-O-, -CH₂-CH₂-CH(CH₃)-,-CH=CH-CH₂-, -CH₂-O-CH₂-O-, -CH₂-NH-O-, -CH₂-N(CH₃)-O-, -CH₂-O-CH₂-, -CH(CH₃)-O-, and -CH(CH₂-O-CH₃)-O-, and/or, -CH₂-CH₂-, and -CH=CH- For all chiral centers, asymmetric groups may be found in either *R* or *S* orientation.

In some embodiments, R^{4*} and R^{2*} together designate the biradical C(R^{a}R^{b})-N(R^{c})-O-, wherein R^{a} and R^{b} are independently selected from the group consisting of hydrogen, halogen, C₁₋₆alkyl, substituted C₁₋₆alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl, such as hydrogen, and; wherein R^{c} is selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ minoalkyl or substituted C₁₋₆ aminoalkyl, such as hydrogen.

In some embodiments, R^{4*} and R^{2*} together designate the biradical C(R^{a}R^{b})-O-C(R^{c}R^{d}) -O-, wherein R^{a}, R^{b}, R^{c}, and R^{d} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl, such as hydrogen.

In some embodiments, R^{4*} and R^{2*} form the biradical -CH(Z)-O-, wherein Z is selected from the group consisting of C₁₋₆ alkyl, C₂₋₆ alkenyl, C₂₋₆ alkynyl, substituted C₁₋₆ alkyl, substituted C₂₋₆ alkenyl, substituted C₂₋₆ alkynyl, acyl, substituted acyl, substituted amide, thiol or substituted thio; and wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ³C(=X)NJ₁J₂ and CN, wherein each J₁, J₂ and J₃ is, independently, H or C₁₋₆alkyl, and X is O, S or NJ₁. In some embodiments Z is C₁₋₆ alkyl or substituted C₁₋₆ alkyl. In some embodiments Z is methyl. In some embodiments Z is substituted C₁₋₆ alkyl. In some embodiments said substituent group is C₁₋₆ alkoxy. In some embodiments Z is CH₃OCH₂-. For all chiral centers, asymmetric groups may be found in either *R* or *S* orientation. Such bicyclic nucleotides are disclosed in US 7,399,845. In some embodiments, R^{1*}, R², R³, R⁵, R^{5*} are hydrogen. In some some embodiments, R^{1*}, R², R^{3*} are hydrogen, and one or both of R⁵, R^{5*} may be other than hydrogen as referred to above and in WO 2007/134181.

In some embodiments, R^{4*} and R^{2*} together designate a biradical which comprise a substituted amino group in the bridge such as consist or comprise of the biradical -CH₂-N( R^{c})-, wherein R^{c} is C₁₋₁₂ alkyloxy. In some embodiments R^{4*} and R^{2*} together designate a biradical -Cq₃q₄-NOR-, wherein q₃ and q₄ are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl; wherein each substituted group is, independently, mono or poly substituted with substituent groups independently selected from halogen, OJ₁, SJ₁, NJ₁J₂, COOJ₁, CN, O-C(=O)NJ₁J₂, N(H)C(=NH)N J₁J₂ or N(H)C(=X=N(H)J₂ wherein X is O or S; and each of J₁ and J₂ is, independently, H, C₁₋₆ alkyl, C₂₋₆ alkenyl, C₂₋₆ alkynyl, C₁₋₆ aminoalkyl or a protecting group. For all chiral centers, asymmetric groups may be found in either *R* or *S* orientation. Such bicyclic nucleotides are disclosed in WO2008/150729. In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. In some embodiments, R^{1*}, R², R³, R⁵, R^{5*} are hydrogen. In some embodiments, R^{1*}, R², R³ are hydrogen and one or both of R⁵, R^{5*} may be other than hydrogen as referred to above and in WO 2007/134181. In some embodiments R^{4*} and R^{2*} together designate a biradical (bivalent group) C(R^{a}R^{b})-O-, wherein R^{a} and R^{b} are each independently halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJ₁ SJ₁, SOJ₁, SO₂J₁, NJ₁J₂, N₃, CN, C(=O)OJ₁, C(=O)NJ₁J₂, C(=O)J₁, O-C(=O)NJ₁J₂, N(H)C(=NH)NJ₁J₂, N(H)C(=O)NJ₁J₂ or N(H)C(=S)NJ₁J₂; or R^{a} and R^{b} together are =C(q3)(q4); q₃ and q₄ are each, independently, H, halogen, C₁-C₁₂alkyl or substituted C₁-C₁₂ alkyl; each substituted group is, independently, mono or poly substituted with substituent groups independently selected from halogen, C₁-C₆ alkyl, substituted C₁-C₆ alkyl, C₂- C₆ alkenyl, substituted C₂-C₆ alkenyl, C₂-C₆ alkynyl, substituted C₂-C₆ alkynyl, OJ₁, SJ₁, NJ₁J₂, N₃, CN, C(=O)OJ₁, C(=O)NJ₁J₂, C(=O)J₁, O-C(=O)NJ₁J₂, N(H)C(=O)NJ₁J₂ or N(H)C(=S)NJ₁J₂, and; each J₁ and J₂ is, independently, H, C1-C₆ alkyl, substituted C1-C₆ alkyl, C₂-C₆ alkenyl, substituted C₂-C₆ alkenyl, C₂-C₆ alkynyl, substituted C₂-C₆ alkynyl, C1-C₆ aminoalkyl, substituted C1-C₆ aminoalkyl or a protecting group. Such compounds are disclosed in WO2009006478A.

In some embodiments, R^{4*} and R^{2*} form the biradical - Q -, wherein Q is C(q₁)(q₂)C(q₃)(q₄), C(q₁)=C(q₃), C[=C(q₁)(q₂)]-C(q₃)(q₄) or C(q₁)(q₂)-C[=C(q₃)(q₄)]; q₁, q₂, q₃, q₄ are each independently. H, halogen, C₁₋₁₂ alkyl, substituted C₁₋₁₂ alkyl, C₂₋₁₂ alkenyl, substituted C₁₋₁₂ alkoxy, OJ₁, SJ₁, SOJ₁, SO₂J₁, NJ₁J₂, N₃, CN, C(=O)OJ₁, C(=O)-NJ₁J₂, C(=O) J₁, -C(=O)NJ₁J₂, N(H)C(=NH)NJ₁J₂, N(H)C(=O)NJ₁J₂ or N(H)C(=S)NJ₁J₂; each J₁ and J₂ is, independently, H, C₁₋₆ alkyl, C₂₋₆ alkenyl, C₂₋₆ alkynyl, C₁₋₆ aminoalkyl or a protecting group; and, optionally wherein when Q is C(q₁)(q₂)(q₃)(q₄) and one of q₃ or q₄ is CH₃ then at least one of the other of q₃ or q₄ or one of q₁ and q₂ is other than H. In some embodiments, R^{1*}, R², R³, R⁵, R^{5*} are hydrogen. For all chiral centers, asymmetric groups may be found in either *R* or *S* orientation. Such bicyclic nucleotides are disclosed in WO2008/154401. In some embodiments, R^{1*}, R², R³, R⁵, R^{5*} are independently selected from the group consisting of hydrogen, halogen, C₁₋₆ alkyl, substituted C₁₋₆ alkyl, C₂₋₆ alkenyl, substituted C₂₋₆ alkenyl, C₂₋₆ alkynyl or substituted C₂₋₆ alkynyl, C₁₋₆ alkoxyl, substituted C₁₋₆ alkoxyl, acyl, substituted acyl, C₁₋₆ aminoalkyl or substituted C₁₋₆ aminoalkyl. In some embodiments, R^{1*}, R², R³, R⁵, R^{5*} are hydrogen. In some embodiments, R^{1*}, R², R³ are hydrogen and one or both of R⁵ R^{5*} may be other than hydrogen as referred to above and in WO 2007/134181 or WO2009/067647 (alpha-L-bicyclic nucleic acids analogs).

In some embodiments the LNA used in the oligonucleotide compounds of the invention preferably has the structure of the general formula II: 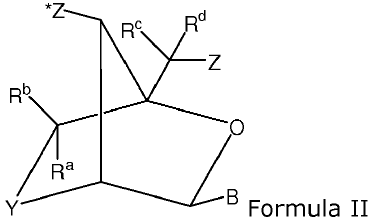 wherein Y is selected from the group consisting of -O-, -CH₂O- -S-, -NH-, N(R^{e}) and/or - CH₂-; Z and Z* are independently selected among an internucleotide linkage, R^{H}, a terminal group or a protecting group; B constitutes a natural or non-natural nucleotide base moiety (nucleobase), and R^{H} is selected from hydrogen and C₁₋₄-alkyl; R^{a}, R^{b} R^{c}, R^{d} and R^{e} are, optionally independently, selected from the group consisting of hydrogen, optionally substituted C₁₋₂-alkyl, optionally substituted C₂₋₁₂-alkenyl, optionally substituted C₂₋₁₂-alkynyl, hydroxy, C₁₋₁₂-alkoxy, C₂-₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂); and R^{H} is selected from hydrogen and C₁₋₄-alkyl. In some embodiments R^{a}, R^{b} R^{c}, R^{d} and R^{e} are, optionally independently, selected from the group consisting of hydrogen and C₁₋₆ alkyl, such as methyl. For all chiral centers, asymmetric groups may be found in either *R* or *S* orientation, for example, two exemplary stereochemical isomers include the beta-D and alpha-L isoforms, which may be illustrated as follows:

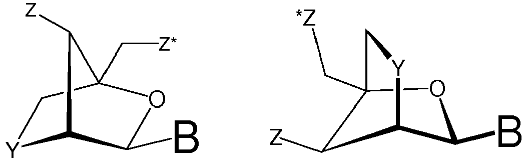

Specific exemplary LNA units are shown below:

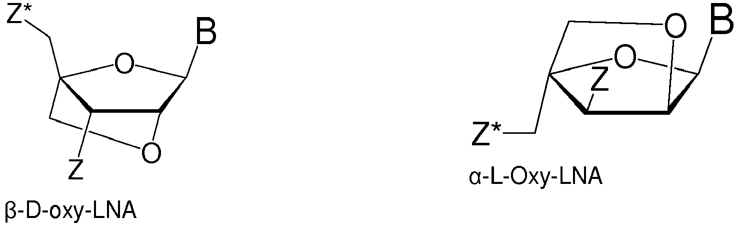

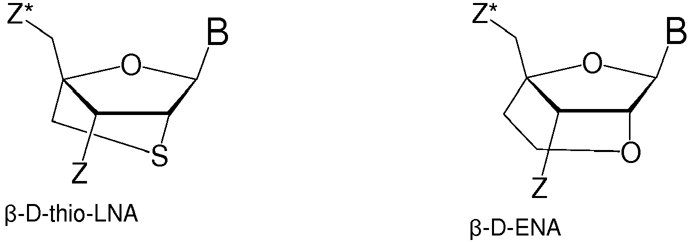

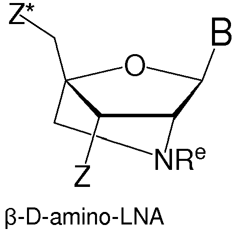

The term "thio-LNA" comprises a locked nucleotide in which Y in the general formula above is selected from S or -CH₂-S-. Thio-LNA can be in both beta-D and alpha-L-configuration.

The term "amino-LNA" comprises a locked nucleotide in which Y in the general formula above is selected from -N(H)-, N(R)-, CH₂-N(H)-, and -CH₂-N(R)- where R is selected from hydrogen and C₁₋₄-alkyl. Amino-LNA can be in both beta-D and alpha-L-configuration.

The term "oxy-LNA" comprises a locked nucleotide in which Y in the general formula above represents -O-. Oxy-LNA can be in both beta-D and alpha-L-configuration.

The term "ENA" comprises a locked nucleotide in which Y in the general formula above is -CH₂-O- (where the oxygen atom of -CH₂-O- is attached to the 2'-position relative to the base B). R^{e} is hydrogen or methyl.

In some exemplary embodiments LNA is selected from beta-D-oxy-LNA, alpha-L-oxy-LNA, beta-D-amino-LNA and beta-D-thio-LNA, in particular beta-D-oxy-LNA.

### Internucleotide Linkages

The terms "linkage group" or "internucleotide linkage" are intended to mean a group capable of covalently coupling together two nucleotides, two nucleotide analogues, and a nucleotide and a nucleotide analogue, etc. Specific and preferred examples include phosphate groups and phosphorothioate groups.

The nucleotides of the oligomer or oligomers or contiguous nucleotides sequence thereof are coupled together via linkage groups. Suitably each nucleotide is linked to the 3' adjacent nucleotide via a linkage group.

Suitable internucleotide linkages include those listed within WO 2007/031091, for example the internucleotide linkages listed on the first paragraph of page 34 of WO 2007/031091 .

It is, in some embodiments, preferred to modify the internucleotide linkage from its normal phosphodiester to one that is more resistant to nuclease attack, such as phosphorothioate or boranophosphate - these two, being cleavable by RNase H, also allow that route of antisense inhibition in reducing the expression of the target gene.

Suitable sulphur (S) containing internucleotide linkages as provided herein may be preferred.

The internucleotide linkages may be selected from, in some embodiments, phosphodiester, phosphorothioate or boranophosphate. Phosphorothioate is preferred, for improved nuclease resistance and other reasons, such as ease of manufacture.

In one aspect of the oligomer or oligomers, the nucleotides and/or nucleotide analogues are linked to each other by means of phosphorothioate groups.

It is recognised that the inclusion of phosphodiester linkages, such as one or two linkages, into an otherwise phosphorothioate oligomer, particularly between or adjacent to nucleotide analogue units can modify the bioavailability and/or bio-distribution of an oligomer - see WO2008/053314.

In some embodiments, the internucleoside linkages of the second oligomer and the first oligomer are phosphorothioate.

In some embodiments, the internucleoside linkages of the second oligomer may comprise one or two phosphodiester linkages, and the remaining internucleotides linkages are phosphorothioate. In some embodiments, which may be the same or different, the internucleoside linkages of the first oligomer may comprise one or two phosphodiester linkages, and the remaining internucleotides linkages are phosphorothioate. In some embodiments, such as the embodiments referred to above, where suitable and not specifically indicated, all remaining linkage groups are either phosphodiester or phosphorothioate, or a mixture thereof.

In some embodiments all the internucleotide linkage groups are phosphorothioate.

In some embodiments the internucleoside linkages between nucleotide analogues are phosphodiester linkages - for example in the mixmer or totalmer oligomers as described herein. Alternatively, phosphorothioate linkages may, in some embodiments, be used. When referring to specific oligomer sequences, such as those provided herein it will be understood that, in various embodiments, when the linkages are phosphorothioate linkages, alternative linkages, such as those disclosed herein may be used, for example phosphate (phosphodiester) linkages may be used, particularly for linkages between nucleotide analogues, such as LNA, units. Likewise, when referring to specific gapmer oligomer sequences, such as those provided herein, when the C residues are annotated as 5'methyl modified cytosine, in various embodiments, one or more of the Cs present in the oligomer may be unmodified C residues.some embodimentsin some embodimentssome embodimentsin some embodiments

### Conjugates

The first and or second oligomer of the invention may be in the form of a conjugate.

In the context the term "conjugate" is intended to indicate a heterogenous molecule formed by the covalent attachment ("conjugation") of the oligomer as described herein to one or more non-nucleotide, or non-polynucleotide moieties. Examples of non-nucleotide or non- polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol.

Therefore, in various embodiments, the oligomer of the invention may comprise both a polynucleotide region which typically consists of a contiguous sequence of nucleotides, and a further non-nucleotide region. When referring to the oligomer of the invention consisting of a contiguous nucleotide sequence, the compound may comprise non-nucleotide components, such as a conjugate component.

In various embodiments of the invention the oligomeric compound is linked to ligands/conjugates, which may be used, e.g. to increase the cellular uptake of oligomeric compounds. WO2007/031091 provides suitable ligands and conjugates (moieties.

The invention also provides for a conjugate comprising the compound according to the invention as herein described, and at least one non-nucleotide or non-polynucleotide moiety covalently attached to said compound. Therefore, in various embodiments where the compound of the invention consists of a specified nucleic acid or nucleotide sequence, as herein disclosed, the compound may also comprise at least one non-nucleotide or non-polynucleotide moiety (e.g. not comprising one or more nucleotides or nucleotide analogues) covalently attached to said compound.

Conjugation (to a conjugate moiety) may enhance the activity, cellular distribution or cellular uptake of the oligomer of the invention. Such moieties include, but are not limited to, antibodies, polypeptides, lipid moieties such as a cholesterol moiety, cholic acid, a thioether, e.g. Hexyl-s-tritylthiol, a thiocholesterol, an aliphatic chain, e.g., dodecandiol or undecyl residues, a phospholipids, e.g., di-hexadecyl-rac-glycerol or triethylammonium 1,2-di-o-hexadecyl-rac-glycero-3-h-phosphonate, a polyamine or a polyethylene glycol chain, an adamantane acetic acid, a palmityl moiety, an octadecylamine or hexylamino-carbonyl-oxycholesterol moiety.

The oligomers of the invention may also be conjugated to active drug substances, for example, aspirin, ibuprofen, a sulfa drug, an antidiabetic, an antibacterial or an antibiotic.

In certain embodiments the conjugated moiety is a sterol, such as cholesterol.

In various embodiments, the conjugated moiety comprises or consists of a positively charged polymer, such as a positively charged peptides of, for example between 1 -50, such as 2 - 20 such as 3 - 10 amino acid residues in length, and/or polyalkylene oxide such as polyethylglycol(PEG) or polypropylene glycol - see WO 2008/034123. Suitably the positively charged polymer, such as a polyalkylene oxide may be attached to the oligomer of the invention via a linker such as the releasable inker described in WO 2008/034123.

By way of example, the following conjugate moieties may be used in the conjugates of the invention:

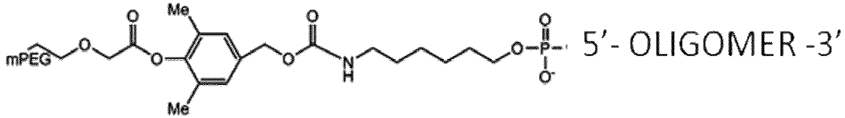

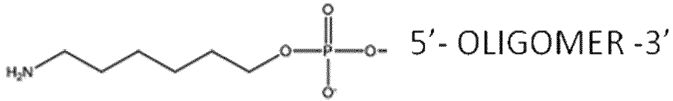

### Activated oligomers

The term "activated oligomer," as used herein, refers to an oligomer of the invention that is covalently linked (i.e., functionalized) to at least one functional moiety that permits covalent linkage of the oligomer to one or more conjugated moieties, i.e., moieties that are not themselves nucleic acids or monomers, to form the conjugates herein described. Typically, a functional moiety will comprise a chemical group that is capable of covalently bonding to the oligomer via, e.g., a 3'-hydroxyl group or the exocyclic NH₂ group of the adenine base, a spacer that is preferably hydrophilic and a terminal group that is capable of binding to a conjugated moiety (e.g., an amino, sulfhydryl or hydroxyl group). In some embodiments, this terminal group is not protected, e.g., is an NH₂ group. In other embodiments, the terminal group is protected, for example, by any suitable protecting group such as those described in "Protective Groups in Organic Synthesis" by Theodora W Greene and Peter G M Wuts, 3rd edition (John Wiley & Sons, 1999). Examples of suitable hydroxyl protecting groups include esters such as acetate ester, aralkyl groups such as benzyl, diphenylmethyl, or triphenylmethyl, and tetrahydropyranyl. Examples of suitable amino protecting groups include benzyl, alpha-methylbenzyl, diphenylmethyl, triphenylmethyl, benzyloxycarbonyl, tert-butoxycarbonyl, and acyl groups such as trichloroacetyl or trifluoroacetyl. In some embodiments, the functional moiety is self-cleaving. In other embodiments, the functional moiety is biodegradable. See e.g., U.S. Patent No. 7,087,229.

In some embodiments, oligomers of the invention are functionalized at the 5' end in order to allow covalent attachment of the conjugated moiety to the 5' end of the oligomer. In other embodiments, oligomers of the invention can be functionalized at the 3' end. In still other embodiments, oligomers of the invention can be functionalized along the backbone or on the heterocyclic base moiety. In yet other embodiments, oligomers of the invention can be functionalized at more than one position independently selected from the 5' end, the 3' end, the backbone and the base.

In some embodiments, activated oligomers of the invention are synthesized by incorporating during the synthesis one or more monomers that is covalently attached to a functional moiety. In other embodiments, activated oligomers of the invention are synthesized with monomers that have not been functionalized, and the oligomer is functionalized upon completion of synthesis. In some embodiments, the oligomers are functionalized with a hindered ester containing an aminoalkyl linker, wherein the alkyl portion has the formula (CH₂)_{w}, wherein w is an integer ranging from 1 to 10, preferably about 6, wherein the alkyl portion of the alkylamino group can be straight chain or branched chain, and wherein the functional group is attached to the oligomer via an ester group (-O-C(O)-(CH₂)_{w}NH).

In other embodiments, the oligomers are functionalized with a hindered ester containing a (CH₂)_{w}-sulfhydryl (SH) linker, wherein w is an integer ranging from 1 to 10, preferably about 6, wherein the alkyl portion of the alkylamino group can be straight chain or branched chain, and wherein the functional group attached to the oligomer via an ester group (-O-C(O)-(CH₂)_{w}SH)

In some embodiments, sulfhydryl-activated oligomers are conjugated with polymer moieties such as polyethylene glycol or peptides (via formation of a disulfide bond).

Activated oligomers containing hindered esters as described above can be synthesized by any method known in the art, and in particular by methods disclosed in PCT Publication No. WO 2008/034122 and the examples therein.

In still other embodiments, the oligomers of the invention are functionalized by introducing sulfhydryl, amino or hydroxyl groups into the oligomer by means of a functionalizing reagent substantially as described in U.S. Patent Nos. 4,962,029 and 4,914,210, i.e., a substantially linear reagent having a phosphoramidite at one end linked through a hydrophilic spacer chain to the opposing end which comprises a protected or unprotected sulfhydryl, amino or hydroxyl group. Such reagents primarily react with hydroxyl groups of the oligomer. In some embodiments, such activated oligomers have a functionalizing reagent coupled to a 5'-hydroxyl group of the oligomer. In other embodiments, the activated oligomers have a functionalizing reagent coupled to a 3'-hydroxyl group. In still other embodiments, the activated oligomers of the invention have a functionalizing reagent coupled to a hydroxyl group on the backbone of the oligomer. In yet further embodiments, the oligomer of the invention is functionalized with more than one of the functionalizing reagents as described in U.S. Patent Nos. 4,962,029 and 4,914,210. Methods of synthesizing such functionalizing reagents and incorporating them into monomers or oligomers are disclosed in U.S. Patent Nos. 4,962,029 and 4,914,210.

In some embodiments, the 5'-terminus of a solid-phase bound oligomer is functionalized with a dienyl phosphoramidite derivative, followed by conjugation of the deprotected oligomer with, e.g., an amino acid or peptide via a Diels-Alder cycloaddition reaction.

In various embodiments, the incorporation of monomers containing 2'-sugar modifications, such as a 2'-carbamate substituted sugar or a 2'-(O-pentyl-N-phthalimido)-deoxyribose sugar into the oligomer facilitates covalent attachment of conjugated moieties to the sugars of the oligomer. In other embodiments, an oligomer with an amino-containing linker at the 2'-position of one or more monomers is prepared using a reagent such as, for example, 5'-dimethoxytrityl-2'-O-(e-phthalimidylaminopentyl)-2'-deoxyadenosine-3'-- N,N-diisopropyl-cyanoethoxy phosphoramidite. See, e.g., Manoharan, et al., Tetrahedron Letters, 1991, 34, 7171.

In still further embodiments, the oligomers of the invention may have amine-containing functional moieties on the nucleobase, including on the N6 purine amino groups, on the exocyclic N2 of guanine, or on the N4 or 5 positions of cytosine. In various embodiments, such functionalization may be achieved by using a commercial reagent that is already functionalized in the oligomer synthesis.

Some functional moieties are commercially available, for example, heterobifunctional and homobifunctional linking moieties are available from the Pierce Co. (Rockford, III.). Other commercially available linking groups are 5'-Amino-Modifier C6 and 3'-Amino-Modifier reagents, both available from Glen Research Corporation (Sterling, Va.). 5'-Amino-Modifier C6 is also available from ABI (Applied Biosystems Inc., Foster City, Calif.) as Aminolink-2, and 3'-Amino-Modifier is also available from Clontech Laboratories Inc. (Palo Alto, Calif.).

### Compositions

The first and second oligomer of the invention may be used in pharmaceutical formulations and compositions. Suitably, such compositions comprise a pharmaceutically acceptable diluent, carrier, salt or adjuvant. WO 2007/031091 provides suitable and preferred pharmaceutically acceptable diluent, carrier and adjuvants. Suitable dosages, formulations, administration routes, compositions, dosage forms, combinations with other therapeutic agents, pro-drug formulations are also provided in WO 2007/031091.

### Kits of the Invention

The kit of the invention comprises of the first oligomer in a first compartment and the second oligomer according to the invention in a second compartment which is independent of the first compartment. The oligomers may be in the form of two independent pharmaceutical compositions.

### Administration

The first oligomer may be provided as a single dose or as a multiple dose - the second oligomer being administered after the first (single dose) or, for example between doses, or after the final dose (multiple dosages of the first oligomer).

The first and second oligomers are administered to the subject who is typically a patient who is suffering from a disease or disorder for which the first oligomer is a suitable therapeutic treatment.

The subject is typically a mammal, such as a rodent (mouse or rat for example), or a primate, preferably a human being.

In some embodiments, the subject is suffering from hyperlipidemia (e.g. when the first oligomer targets ApoB or PCSK9 or has-miR122).

In some embodiments, the subject is suffering from cancer (e.g. when the first oligomer targets Hif1alpha, survive, Bcl2, Mcl1, Her3, androgen receptor, beta-catenin, Pl3 kinase, or FABP4 etc.)

In some embodiments, the subject is suffering from an inflammaotry disease, such as arthritis (e.g. when the first oligomer targets TNFR-alpha).

In some embodiments, the subject is suffering from an infectious disease, such as Hepatitis C infection (e.g. when the first oligomer targets miR122).

The invention therefore further comprises methods for treating the disease or disorders referred to herein, or for the use of the kit of the invention or the first and second oligomers for treating the disease or disorders referred to herein. In an even further aspect, the present invention relates to the use of the second oligomer according to the invention for use in the treatment of, or for use in regulating the treatment of, a disease or disorder in the subject, such as a disease or disorder selected from the group consisting of: atherosclerosis, hypercholesterolemia and hyperlipidemia; cancer, glioblastoma, breast cancer, lymphoma, lung cancer; diabetes, metabolic disorders; myoblast differentiation; immune disorders. In some embodiments, the disease or disorder is a disease selected from the group consisting of: atherosclerosis, hypercholesterolemia and hyperlipidemia (e.g in the case of ApoB, PSCK or FABP4 targeting first oligomers); cancer, glioblastoma, breast cancer, lymphoma, lung cancer; diabetes, metabolic disorders; myoblast differentiation; immune disorders.

It is recognised that the specific tissue biodistribution pattern of an oligomer in the subject depends upon he specific chemistry and possibly the sequence of the oligomer, and as such this feature of oligonucleotide therapeutics may be exploited to specifically target the first oligomer is specific tissues where its activity may be detrimental, whilst allowing the therapeutic benefit of the first oligomer to proceed in the tissues or cells where is therapeutic activity are most desirable and effective. The biodistribution of the first and second oligomer within the subject may, in some embodiments, differ - this can be achieved, for example, by the insertion of one or two phosphodiester bonds into a phosphorothioate backbone, which is known to cause an enhanced accumulation of the oligomer in kidney cells. In such as instance, if the first oligomer targets a RNA target in, for, example in the liver, the use of a partial phosphodiester/phosphorothioate second oligomer can preferentially alleviate the effect of the first oligomer in the kidney - for example in the instance that the first oligomer causes undesirable effects in the kidney such as is toxic to kidney cells. The method according to the iinvention, therefore, provides a method for selectively reducing the bioavailability of a first oligomer in a tissue, such as the kidney, or, in other words, a method for reducing the toxicity of the first oligomer in a specific tissue. In some embodiments this may allow the use of a highly effective first oligomer for a therapeutic treatment, despite the toxicity of the first oligomer in specific (e.g. non-target) tissues or cells in the subject.

Indeed, the use of the second oligomer may be used for the treatment of toxicity associated with the use of oligonucleotide therapeutics, such as the first oligomer, for example in the treatment of (e.g therapeutic oligonucleotide induced) hepatic steosis - allowing for the reversal of the hepatic steosis and a return to normal liver morphology.

Typical dosages of the first and/or second oligomer are between 0.1 and 25 mg/kg depending on the oligo design and potency. In some embodiments the dose regimen may be between daily to weekly dosing.

The administration may be via any suitable administration route, such as iv, ip, sc, oral, for example.

For the first oligomer, typically there is a time period of between 1 day to 2 months between dosages, such as between 2 days and 1 month. The dosage regimen may be for example every day, every other day, every third day, every fourth day, every fifth day, every sixth day, or every week, or every two weeks, or every three weeks or every month. The second oligomer may be administered after the first or after several administrations of the first oligomer. The second oligomer may also be provided in single or multiple dosages - suitably the bioavailability of the first oligomer or the phenotypic effects of the first oligomer may be monitored after the administration of the second oligomer, and if necessary further dosages of the second oligomer may then be provided.

In some embodiments the dosages of the first and second oligomer are, for each individual dose, provided at the same dosage level (i.e. a ration of 1:1). However in some other embodiments, the second oligomer is administered at a higher dosage than the first oligomer. It is recognized that the dosage of the second oligomer should be optimized to provide an effective antidote effect without causing detrimental side effects itself. In this regards, the design of the second oligomer is an important variable in that by ensuring at least one or more, even all the nucleotide analogues of the first and second oligomers align when the first and second oligomer form a duplex (*i.e.* the nucleotide analogues are in corresponding positions) - the second oligomer (the antidote) can be designed so that it has a very high specificity and affinity towards the first olgiomer. By matching the positions of affinity enhancing nucleotides in the first and second oligomers, it is also possible to reduce the length of the second oligomer, ensuring a low or even negligible affinity to off target sequences - in this regard it is considered that, in some embodiments, the second oligomer may be shorter in length (*i.e.* the length of the respective contiguous nucleotide sequences) than the first oligomer.

Suitably, the time between the administration of the first oligomer and the second oligomer are such that the first oligomer is present in the subject, such as is present at detectable levels in the subject immediately prior to the administration of the second oligomer. The time period will be dependent upon a number of factors including the time required for the first oligomer to elicit its therapeutic effect, the time at which deleterious side-effects In some embodiments the time between the administration of the first oligomer and the second oligomer may be between 30 minutes to 2 months, such as between 1 hour and 1 month. In some embodiments the time period is at least 30 minutes or at least 1 hour, such as at least 6 hours, such as at least 12 hours, such as at least 24 hours, such as at least 2 or 3 days, such as at least 1 week. In some embodiments the time period is no longer than 2 months, such as no longer than 6 weeks, such as no longer than 1 month, such as no longer than 3 weeks, such as no longer than 2 weeks, such as no longer than 1 week.

### EXAMPLES

### Example 1: Monomer synthesis

The LNA monomer building blocks and derivatives are prepared following the procedures disclosed in WO07/031081 and the references cited therein.

### Example 2: Oligonucleotide synthesis

Oligonucleotides are synthesized according to the method described in WO07/031081.

### Example 3: Naked Oligo Treatment with Antisense Antidote kit:

1. Cells are seeded in 6-well plates at an appropriate cell density the day before oligo treatment.
2. Cell plating has to be optimized for different cell lines..
3. On the day 0 of antisense treatment, prepare fresh oligo solution by diluting stock oligo solution in complete media. For example, to make 2 mL of 5 µM solution, add 20 µL of a 500 µM stock to 1980 µL of complete media. Please note that 2.5 to 5 µM would be a reasonable concentration range to start with. Depending on the cell line and the target of the oligo, you may have to adjust the concentrations appropriately.
4. Control sample would be the untreated cells and cells treated either only with antisense or antidote. There will be no mock since no transfection reagent is used.
5. When all oligo solutions are prepared, remove the media in each well and add 2 mL of the naked oligo solutions (for control sample, add 2 mL of complete media). Then, incubate in 37°C and 5% CO₂ incubator.
6. On the day e.g. 4 of antidote treatment, prepare fresh oligo solution by diluting stock antidote oligo solution in complete media. For example, to make 2 mL of 10 µM solution, add 20 µL of a 1 mM stock to 1980 µL of complete media. Replace the antisense media in all well except controls with antidote media. Place cells in 37°C and 5% CO₂ incubator.
7. The time of incubation is dependent on different experiments. However, oligo solutions should be left in the wells for the whole duration of treatment. A general guideline for the incubation time would be 3 to 6 days after initial treatment.
8. For qPCR, rinse cells with PBS and harvest them by adding 350 mL of RLT buffer from RNeasy Mini kit. Then, follow the standard procedure for preparing and purifying total RNA.
9. For Western blotting, rinse cells with PBS and harvest by scraping the cells with a cell scraper. Follow the standard procedure for Western blotting.

### Example 4: Example Antidote designs:

Antidotes are designed to be biostable and to preferentially bind the antisense LNA drug over other nucleotide sequences preferable by LNA:LNA hybridisation between antisense and antidote. Antidotes can be phosphorothioates but also phosphodiester or combination of the two. Antidotes are designed not to recruit RNaseH and typically do not comprise more than 3, such as more than two consecutive DNA residues.

**Table of selected first oligomers and antidote oligomers:**

| **Target** | **Antisense** | **Antidote** |
|---|---|---|
| ApoB | 5'-GCattggtatTCA-3' (SEQ 1) | 3'-CGtAaCcAtAaGT 5' (SEQ 2) |
| HER3 | 5'-GTGtgtgtatttcCCA-3' (SEQ 3) | 3' CaCaCaCaTaAaGggT-5' (SEQ 4) |
| Hif1 alpha | 5'-TGGcaagcatccTGTa-3' (SEQ 5) | 3'- AcCgTtCgTaGgAcAT 5' (SEQ 6) |
| Hif1 alpha | 5'-GCaagcatccTG-3' (SEQ 7) | 3'-CGtTcGtAgGAC-5' (SEQ 8) |
| miR122 | 5'-CcAttGTcaCaCtCC-3' (SEQ 9) | 3'-GgTaaCAgtGtGaGG-5' (SEQ 10) |
| miR-19b | 5'-TgCatGGatTtGcAC-3' (SEQ 11) | 3'-AcGtaCCtaAaCgTG-5' (SEQ 12) |
| miR-155 | 5'-TcAcgATtaGcAtTA-3' (SEQ 13) | 3'-AgTgcTAatCgTaAT-5' (SEQ 14) |
| miR-21 | 5'- TcAgtCTgaTaAgCT -3' (SEQ 15) | 3'-AgTcaGActAtTcGA -5' (SEQ 16) |
| miR-375 | 5'- GtGccGTtcGtTcTT 3' (SEQ 17) | 3'-CaCggCAagCaAgAA-5' (SEQ 18) |

*Capital letters = LNA nucleotides (e.g. beta-D-oxy LNA). Small letters = DNA nucleotides. Internucleoside linkages are phosphorothioate. LNA cytosines are preferably 5'-methyl.*

### Example 5: Animal experiment

The experiments were performed according to the principles stated in the Danish law on animal experiments and were approved by the Danish Animal Experiments Inspectorate, Ministry of Justice, Denmark. Housing: Inbred female C57BU6J female mice were obtained from Taconic M&B (Denmark) and fed ad libitum a commercially pelleted mouse diet (altromin no 1324, Denmark) containing approximately 4 wt% fat. The animal room was illuminated to give a cycle of 12 hours light and 12 hours darkness and temperature control was 21 °C ± 2°C and relative humidity 55 ± 10%. Treatment and sampling: LNA oligonucleotides were administered to the mice based upon body weight by tail vein injections (10 ml/kg). The test compounds were formulated in saline and saline (0.9 % NaCl) was used as control. The LNA oligonucleotides were administered 1 or 5 mg/kg/dose. Dosing may be performed more than once, such as on days 0, 2 and 4 or weekly.

### Example 6: Total RNA extraction

Dissected livers from sacrificed mice were immediately stored in RNA later (Ambion). Approximately 30 mg of tissue was added metal spheres (Qiagen) and RLT lysis buffer (Qiagen) and homogenized for 5 min. Supernatants of the liver homogenate was added 1:1 ration of cold 70% ethanol and total RNA was purified using the spin column method of RNeasy mini kit (Qiagen) according to the manufacturer's instructions.

### Example 7: Quantitative RT-PCR

mRNA quantification of selected genes was done using commercially available TaqMan assays (Applied Biosystems). First strand cDNA was generated from total RNA by reverse transcription reaction using random decamers, 0.5 µg total RNA, and the M-MLV RT enzyme (Ambion) according to manufacturer's instructions. The cDNA was subsequently diluted 10 times in nuclease-free water before addition to the RT-PCR reaction mixture. A two-fold cDNA dilution series from liver total RNA of a saline-treated animal or mock transfected cells cDNA reaction (using 2.5 times more total RNA than in samples) served as standard to ensure a linear range (Ct versus relative copy number) of the amplification. Applied Biosystems 7500Fast real-time PCR instrument was used for amplification. Data was analyzed and quantified using the 7500Fast SDS software. ApoB mRNA levels were normalized to GAPDH mRNA and plotted relative to saline or mock treated groups.

### Example 8: Cholesterol levels in serum

Total cholesterol level is measured in serum using a colometric assay Cholesterol CP from ABX Pentra. The cholesterol is measured following enzymatic hydrolysis and oxidation. 21.5 µL water was added to 1.5 µL plasma. 250 µL reagent is added and within 5 min the cholesterol content is measured at a wavelength of 540 nM. Measurements on each animal were made in duplicates. The sensitivity and linearity was tested with 2 fold diluted control compound (ABX Pentra N control). The relative Cholesterol level was determined by subtraction of the background and presented relative to the cholesterol levels in plasma of saline treated mice.

### Example 9: ALT in serum

The activity of alanine-aminotransferase (ALT) in mouse serum was determined using an enzymatic ALT assay from Horiba ABX Diagnostics (Triolab) according to the manufacturer's instruction but adjusted to 96-well format. In brief, serum samples were diluted 2.5 fold with H₂O and assayed in duplicate. After addition of 50 µl diluted sample or multical standard from Horiba ABX Diagnostics (Triolab) to each well, 200 µl of 37°C ALT reagent mix was added to each well. Kinetic measurements were performed at 340nm and 37°C for 5 min with an interval of 30 sek. Data were correlated to the 2-fold diluted standard curve and results were presented as ALT activity in U/L.

### Example 10: Sense antidote effect of SEQ ID NO: 2 on apoB mRNA and total cholesterol levels in C57BL/6 female mice treated with SEQ ID NO: 1.

To evaluate the sense antidote effect of SEQ ID NO: 2 on SEQ ID NO: 1 C57BL/6 female mice were administered 1 mg/kg of SEQ ID NO: 1, 5 mg/kg SEQ ID NO: 2 or saline on day 0, 2 and 4. One hour after last dosing mice treated with SEQ ID NO: 1 was administered 5 mg/kg of sense antidote SEQ ID NO: 2. Mice from all groups were sacrificed on day 5 and 8. Whole blood was sampled on day 4, 5 and 8. Liver tissues were analysed for apoB mRNA expression and serums for ALT and total cholesterol.

The results showed that mice treated with SEQ ID NO: 1 alone obtained continuous reduction in apoB throughout the entire study to a maximum reduction of ∼70% left relative to saline treated mice. No effect on apoB mRNA was seen in livers of mice treated with the sense antidote SEQ ID NO: 2 alone. The group of mice given sense antidote SEQ ID NO: 2 after treatment with SEQ ID NO: 1 showed increase in apoB mRNA compared to SEQ ID NO: 1 alone treated mice at day 5 and complete reversed to saline level at day 8. Total serum cholesterol levels followed the same pattern as for apoB mRNA levels and no significant increase in serum ALT was seen in any of the treatment groups.
31. The antidote compound of any of the above embodiments, wherein the antidote compound activates RNase H.
32. The antidote compound of any of the above embodiments, wherein the antidote compound activates the RISC pathway.
33. A composition comprising an antidote compound according to any of the above embodiments or a salt thereof and a pharmaceutically acceptable carrier or diluent.
34. A method comprising administering to an animal a compound or composition according to any of the above embodiments.
35. The method of embodiment 34, wherein the animal is a human.
36. The method of embodiment 35, wherein the administering is oral, topical, or parenteral.
37. A method of inhibiting antisense activity in a cell comprising contacting the cell with an antidote compound according to any of the above embodiments and thereby inhibiting the antisense activity in the cell.
38. The method of embodiment 37, wherein the cell is in an animal.
39. The method of embodiment 38, wherein the animal is a human.
40. A method comprising: contacting a cell with an antisense compound; detecting antisense activity; and contacting the cell with an antidote compound.
41. The method of embodiment 40, wherein the antidote compound is any antidote compound of embodiments 1 to 32.
42. The method of embodiment 40, wherein the detecting antisense activity comprises measuring the amount of target mRNA present.
43. The method of embodiment 40, wherein the detecting antisense activity comprises measuring the amount of target protein present.
44. The method of embodiment 40, wherein the detecting antisense activity comprises measuring activity of a target protein.
45. The method of any of embodiments 40 to 44 comprising detecting antidote activity by measuring antisense activity after contacting the cell with antidote compound.
46. The method of any of embodiments 40 to 45 wherein the cell is in an animal.
47. The method of embodiment 46, wherein the animal is a human.
48. A method of ameliorating a side-effect of antisense treatment comprising: contacting a cell with an antisense compound; detecting a side-effect; contacting the cell with an antidote compound; and thereby ameliorating the side effect of the antisense compound.
49. The method of embodiment 40, wherein the antidote compound is any antidote compound of embodiments 1 to 32.
50. A method of treating a patient comprising: administering to the patient an antisense compound; monitoring the patient for antisense activity; and if the antisense activity becomes higher than desired, administrating an antidote compound.
51. The method of embodiment 50, wherein the antidote compound is any antidote compound of embodiments 1 to 32.
52. The method of embodiment 50, wherein the monitoring antisense activity comprises measuring the amount of target mRNA present.
53. The method of embodiment 50, wherein the monitoring antisense activity comprises measuring the amount of target protein present.
54. The method of embodiment 50, wherein the monitoring antisense activity comprises measuring activity of a target protein.
55. The method of any of embodiments 50 to 54 comprising detecting antidote activity by measuring antisense activity after administration of the antidote compound.
56. The method of any of embodiments 50 to 55 wherein the patient is a human.
57. A method of treating a patient comprising: administering to the patient an antisense compound; monitoring the patient for one or more side effect; and if the one or more side effect reaches an undesirable level, administrating an antidote compound.
58. The method of embodiment 57, wherein the antidote compound is any antidote compound of embodiments 1 to 32.
59. The method of any of embodiments 57 to 58 wherein the patient is a human.
60. A kit comprising an antisense compound and an antidote compound.
61. A kit comprising an antidote compound and a non-oligomeric antidote.
62. The kit of embodiment 61 wherein the non-oligomeric antidote is a target protein.

### EXAMPLES

### Example 1: Monomer synthesis

The LNA monomer building blocks and derivatives are prepared following the procedures disclosed in WO07/031081 and the references cited therein.

### Example 2: Oligonucleotide synthesis

Oligonucleotides are synthesized according to the method described in WO07/031081.

### Example 3: Naked Oligo Treatment with Antisense Antidote kit:

1. Cells are seeded in 6-well plates at an appropriate cell density the day before oligo treatment.
2. Cell plating has to be optimized for different cell lines..
3. On the day 0 of antisense treatment, prepare fresh oligo solution by diluting stock oligo solution in complete media. For example, to make 2 mL of 5 µM solution, add 20 µL of a 500 µM stock to 1980 µL of complete media. Please note that 2.5 to 5 µM would be a reasonable concentration range to start with. Depending on the cell line and the target of the oligo, you may have to adjust the concentrations appropriately.
4. Control sample would be the untreated cells and cells treated either only with antisense or antidote. There will be no mock since no transfection reagent is used.
5. When all oligo solutions are prepared, remove the media in each well and add 2 mL of the naked oligo solutions (for control sample, add 2 mL of complete media). Then, incubate in 37°C and 5% CO₂ incubator.
6. On the day e.g. 4 of antidote treatment, prepare fresh oligo solution by diluting stock antidote oligo solution in complete media. For example, to make 2 mL of 10 µM solution, add 20 µL of a 1 mM stock to 1980 µL of complete media. Replace the antisense media in all well except controls with antidote media. Place cells in 37°C and 5% CO₂ incubator.
7. The time of incubation is dependent on different experiments. However, oligo solutions should be left in the wells for the whole duration of treatment. A general guideline for the incubation time would be 3 to 6 days after initial treatment.
8. For qPCR, rinse cells with PBS and harvest them by adding 350 mL of RLT buffer from RNeasy Mini kit. Then, follow the standard procedure for preparing and purifying total RNA.
9. For Western blotting, rinse cells with PBS and harvest by scraping the cells with a cell scraper. Follow the standard procedure for Western blotting.

### Example 4: Example Antidote designs:

Antidotes are designed to be biostable and to preferentially bind the antisense LNA drug over other nucleotide sequences preferable by LNA:LNA hybridisation between antisense and antidote. Antidotes can be phosphorothioates but also phosphodiester or combination of the two. Antidotes are designed not to recruit RNaseH and typically do not comprise more than 3, such as more than two consecutive DNA residues.

**Table of selected first oligomers and antidote oligomers:**

| **Target** | **Antisense** | **Antidote** |
|---|---|---|
| ApoB | 5'-GCattggtatTCA-3' (SEQ 1) | 3'-CGtAaCcAtAaGT 5' (SEQ 2) |
| HER3 | 5'-GTGtgtgtatttcCCA-3' (SEQ 3) | 3' CaCaCaCaTaAaGggT-5' (SEQ 4) |
| Hif1 alpha | 5'-TGGcaagcatccTGTa-3' (SEQ 5) | 3'- AcCgTtCgTaGgAcAT 5' (SEQ 6) |
| Hif1 alpha | 5'-GCaagcatccTG-3' (SEQ 7) | 3'-CGtTcGtAgGAC-5' (SEQ 8) |
| miR122 | 5'-CcAttGTcaCaCtCC-3' (SEQ 9) | 3'-GgTaaCAgtGtGaGG-5' (SEQ 10) |
| miR-19b | 5'-TgCatGGatTtGcAC-3' (SEQ 11) | 3'-AcGtaCCtaAaCgTG-5' (SEQ 12) |
| miR-155 | 5'-TcAcgATtaGcAtTA-3' (SEQ 13) | 3'-AgTgcTAatCgTaAT-5' (SEQ 14) |
| miR-21 | 5'- TcAgtCTgaTaAgCT -3' (SEQ 15) | 3'-AgTcaGActAtTcGA -5' (SEQ 16) |
| miR- 375 | 5'- GtGccGTtcGtTcTT 3' (SEQ 17) | 3'-CaCggCAagCaAgAA-5' (SEQ 18) |

*Capital letters = LNA nucleotides (e.g. beta-D-oxy LNA). Small letters = DNA nucleotides. Internucleoside linkages are phosphorothioate. LNA cytosines are preferably 5'-methyl.*

### Example 5: Animal experiment

The experiments were performed according to the principles stated in the Danish law on animal experiments and were approved by the Danish Animal Experiments Inspectorate, Ministry of Justice, Denmark. Housing: Inbred female C57BL/6J female mice were obtained from Taconic M&B (Denmark) and fed ad libitum a commercially pelleted mouse diet (altromin no 1324, Denmark) containing approximately 4 wt% fat. The animal room was illuminated to give a cycle of 12 hours light and 12 hours darkness and temperature control was 21 °C ± 2°C and relative humidity 55 ± 10%. Treatment and sampling: LNA oligonucleotides were administered to the mice based upon body weight by tail vein injections (10 ml/kg). The test compounds were formulated in saline and saline (0.9 % NaCl) was used as control. The LNA oligonucleotides were administered 1 or 5 mg/kg/dose. Dosing may be performed more than once, such as on days 0, 2 and 4 or weekly.

### Example 6: Total RNA extraction

Dissected livers from sacrificed mice were immediately stored in RNA later (Ambion). Approximately 30 mg of tissue was added metal spheres (Qiagen) and RLT lysis buffer (Qiagen) and homogenized for 5 min. Supernatants of the liver homogenate was added 1:1 ration of cold 70% ethanol and total RNA was purified using the spin column method of RNeasy mini kit (Qiagen) according to the manufacturer's instructions.

### Example 7: Quantitative RT-PCR

mRNA quantification of selected genes was done using commercially available TaqMan assays (Applied Biosystems). First strand cDNA was generated from total RNA by reverse transcription reaction using random decamers, 0.5 µg total RNA, and the M-MLV RT enzyme (Ambion) according to manufacturer's instructions. The cDNA was subsequently diluted 10 times in nuclease-free water before addition to the RT-PCR reaction mixture. A two-fold cDNA dilution series from liver total RNA of a saline-treated animal or mock transfected cells cDNA reaction (using 2.5 times more total RNA than in samples) served as standard to ensure a linear range (Ct versus relative copy number) of the amplification. Applied Biosystems 7500Fast real-time PCR instrument was used for amplification. Data was analyzed and quantified using the 7500Fast SDS software. ApoB mRNA levels were normalized to GAPDH mRNA and plotted relative to saline or mock treated groups.

### Example 8: Cholesterol levels in serum

Total cholesterol level is measured in serum using a colometric assay Cholesterol CP from ABX Pentra. The cholesterol is measured following enzymatic hydrolysis and oxidation. 21.5 µL water was added to 1.5 µL plasma. 250 µL reagent is added and within 5 min the cholesterol content is measured at a wavelength of 540 nM. Measurements on each animal were made in duplicates. The sensitivity and linearity was tested with 2 fold diluted control compound (ABX Pentra N control). The relative Cholesterol level was determined by subtraction of the background and presented relative to the cholesterol levels in plasma of saline treated mice.

### Example 9: ALT in serum

The activity of alanine-aminotransferase (ALT) in mouse serum was determined using an enzymatic ALT assay from Horiba ABX Diagnostics (Triolab) according to the manufacturer's instruction but adjusted to 96-well format. In brief, serum samples were diluted 2.5 fold with H₂O and assayed in duplicate. After addition of 50 µl diluted sample or multical standard from Horiba ABX Diagnostics (Triolab) to each well, 200 µl of 37°C ALT reagent mix was added to each well. Kinetic measurements were performed at 340nm and 37°C for 5 min with an interval of 30 sek. Data were correlated to the 2-fold diluted standard curve and results were presented as ALT activity in U/L.

### Example 10: Sense antidote effect of SEQ ID NO: 2 on apoB mRNA and total cholesterol levels in C57BL/6 female mice treated with SEQ ID NO: 1.

To evaluate the sense antidote effect of SEQ ID NO: 2 on SEQ ID NO: 1 C57BL/6 female mice were administered 1 mg/kg of SEQ ID NO: 1, 5 mg/kg SEQ ID NO: 2 or saline on day 0, 2 and 4. One hour after last dosing mice treated with SEQ ID NO: 1 was administered 5 mg/kg of sense antidote SEQ ID NO: 2. Mice from all groups were sacrificed on day 5 and 8. Whole blood was sampled on day 4, 5 and 8. Liver tissues were analysed for apoB mRNA expression and serums for ALT and total cholesterol.

The results showed that mice treated with SEQ ID NO: 1 alone obtained continuous reduction in apoB throughout the entire study to a maximum reduction of ∼70% left relative to saline treated mice. No effect on apoB mRNA was seen in livers of mice treated with the sense antidote SEQ ID NO: 2 alone. The group of mice given sense antidote SEQ ID NO: 2 after treatment with SEQ ID NO: 1 showed increase in apoB mRNA compared to SEQ ID NO: 1 alone treated mice at day 5 and complete reversed to saline level at day 8. Total serum cholesterol levels followed the same pattern as for apoB mRNA levels and no significant increase in serum ALT was seen in any of the treatment groups.

## Claims

1. An antidote compound, the second oligomer, comprising a single stranded oligomer 6 - 30 contiguous nucleotides in length with at least one nucleotide analogue, for use as a medicament for deactivation of a therapeutic first oligomer *in vivo* in a subject, wherein
a) the first oligomer is complementary or essentially complementary to a target nucleic acid naturally present in the subject and the first oligomer comprise one or more nucleotide analogues selected from locked nucleic acid (LNA), 2'-O-alkyl-RNA, 2'-OMe-RNA, 2'-amino-DNA, 2'-fluoro-DNA, 2'-MOE-RNA; and
b) the sequence of contiguous nucleotides of said second oligomer is i) completely complementary to a sequence of contiguous nucleotides present in the first oligomer over the length of the shorter of the two oligomers or ii) comprises no more than a single mismatch with the compliment of a sequence of contiguous nucleotides present in the first oligomer over the length of the shorter of the two oligomers and said second oligomer comprise one or more nucleotide analogues selected from LNA, 2'-O-alkyl-RNA, 2'-OMe-RNA, 2'-amino-DNA, 2'-fluoro-DNA, 2'-MOE-RNA and is designed so that the duplex between the first oligomer and the complementary second oligomer results in the formation of at last one nucleotide analogue base pair; and
c) the second oligomer does not comprise a contiguous sequence of more than 3 DNA units.

2. The antidote compound for use as a medicament according to claim 1, wherein the 5' and 3' nucleotides in the second oligomer is LNA nucleotides.

3. The antidote compound for use as a medicament according to claim 1 or 2, wherein the nucleotide analogue pair formed between the first and second oligomer is selected from LNA/LNA, LNA/2'MOE, LNA/2'fluoro, 2'MOE/LNA or 2'fluoro/LNA (first oligomer/second oligomer).

4. The antidote compound for use as a medicament according to any one of claims 1 - 3, wherein the first oligomer has a contiguous nucleotide sequence which is either i) fully complementary to a sub-sequence of contiguous nucleotides present in a mammalian RNA target, or ii) comprises no more than a single mismatch with the complement of a sub-sequence of contiguous nucleotides present in said RNA target.

5. The antidote compound for use as a medicament according to any one of claims 1 - 4, wherein said contiguous nucleotide sequence of said first oligomer has a length of between 8 and 30 nucleotides.

6. The antidote compound for use as a medicament according to any one of claims 1 - 5, wherein said first oligomer is a gapmer oligonucleotide.

7. The antidote compound for use as a medicament according to any one of claims 1- 6, wherein said RNA target is a human mRNA, such as a mRNA selected from the group consisting of ApoB-100, CRP, PCSK9, PTP-1 B, GCGR, GCCR, SGLT2, clusterin, survivin, eiF-4E, Hsp27, ICAM-1, VLA-4, IL-4Ralpha, C-ref kinase, SOD1, and GHr.

8. The antidote compound for use as a medicament according to any one of claims 1 - 7, wherein the first oligomer targets PCSK9 or ApoB and the first oligomer treats a disease selected from the group consisting of atherosclerosis, hypercholesterolemia and hyperlipidemia.

9. The antidote compound for use as a medicament according to any one of claims 1 - 7, wherein the first oligomer targets Hif1 alpha, survivin, Bcl2, Mcl1, Her3, androgen receptor, beta-catenin, PI3 kinase, or FABP4, and the first oligomer treats cancer.

10. The antidote compound for use as a medicament according to any one of claims 1 - 5, wherein said RNA target is a human microRNA.

11. The antidote compound for use as a medicament according to any one of claims 1 to 5 or claim 10 wherein the first oligomer targets miR-122, and, wherein the first oligomer treats Hepatitis C infection.

12. The antidote compound for use as a medicament according to any one of claims 1 -11, wherein the second oligomer is a mixmer which comprises both naturally occurring nucleosides and LNA nucleotide analogues.

13. The antidote compound for use as a medicament according to any one of claims 1 - 11, wherein the second oligomer is a totalmer where all of the nucleotides are nucleotide analogues selected from LNA, 2'-O-alkyl-RNA, 2'-OMe-RNA, 2'-amino-DNA, 2'-fluoro-DNA, 2'-MOE-RNA.

14. The antidote compound for use as a medicament according to any one of claims 1 - 13, wherein the internucleoside linkages present in the second oligomer are phosphorothioate linkages.

15. A kit for therapeutic use, comprising a first oligomer in a first compartment and a second oligomer in a second compartment, wherein the oligomers are defined according to any one of claims 1-14.

## Patentansprüche

1. Antidotverbindung, das zweite Oligomer, umfassend ein einsträngiges Oligomer mit einer Länge von 6 - 30 zusammenhängenden Nukleotiden mit mindestens einem Nukleotidanalogon zur Verwendung als Arzneimittel zur Deaktivierung eines therapeutischen ersten Oligomers *in vivo* bei einem Patienten, wobei
a) das erste Oligomer komplementär oder im Wesentlichen komplementär zu einer Ziel-Nukleinsäure ist, die natürlich bei dem Patienten vorkommt, und das erste Oligomer ein oder mehrere Nukleotidanaloga umfasst, ausgewählt aus LNA (Locked Nucleic Acid), 2'-O-Alkyl-RNA, 2'-OMe-RNA, 2'-Amino-DNA, 2'-Fluor-DNA, 2'-MOE-RNA; und
b) die Sequenz zusammenhängender Nukleotide des zweiten Oligomers i) vollständig komplementär zu einer Sequenz zusammenhängender Nukleotide ist, die in dem ersten Oligomer über eine Länge des kürzeren der zwei Oligomere vorhanden sind, oder ii) nicht mehr als ein einziges Mismatch mit dem Komplement einer Sequenz zusammenhängender Nukleotide aufweist, die in dem ersten Oligomer über eine Länge des kürzeren der zwei Oligomere vorhanden sind, und wobei das zweite Oligomer ein oder mehrere Nukleotidanaloga aufweist, ausgewählt aus LNA, 2'-O-Alkyl-RNA, 2'-OMe-RNA, 2'-Amino-DNA, 2'-Fluor-DNA, 2'-MOE-RNA, und so beschaffen ist, dass das Duplex zwischen dem ersten Oligomer und dem komplementären zweiten Oligomer zur Bildung mindestens eines Nukleotidanalogon-Basenpaares führt; und
c) das zweite Oligomer keine zusammenhängende Sequenz von mehr als drei DNA-Einheiten umfasst.

2. Antidotverbindung zur Verwendung als Arzneimittel nach Anspruch 1, wobei das 5'- und das 3'-Nukleotid des zweiten Oligomers ein LNA-Nukleotid ist.

3. Antidotverbindung zur Verwendung als Arzneimittel nach Anspruch 1 oder 2, wobei das zwischen dem ersten und dem zweiten Oligomer ausgebildete Nukleotidanalogon-Paar ausgewählt ist aus LNA/LNA, LNA/2'MOE, LNA/2'Fluor, 2'MOE/LNA oder 2'Fluor/LNA (erstes Oligomer/zweites Oligomer).

4. Antidotverbindung zur Verwendung als Arzneimittel nach einem der Ansprüche 1 - 3, wobei das erste Oligomer eine zusammenhängende Nukleotidsequenz aufweist, die entweder i) vollständig mit einer Untersequenz in einem Säugetier-RNA-Ziel vorhandener zusammenhängender Nukleotide komplementär ist oder ii) nicht mehr als ein einziges Mismatch mit dem Komplement einer Untersequenz in dem RNA-Ziel vorhandener zusammenhängender Nukleotide aufweist.

5. Antidotverbindung zur Verwendung als Arzneimittel nach einem der Ansprüche 1 - 4, wobei die zusammenhängende Nukleotidsequenz des ersten Oligomers eine Länge von 8 bis 30 Nukleotiden aufweist.

6. Antidotverbindung zur Verwendung als Arzneimittel nach einem der Ansprüche 1 - 5, wobei das erste Oligomer ein Gapmer-Oligonukleotid ist.

7. Antidotverbindung zur Verwendung als Arzneimittel nach einem der Ansprüche 1 - 6, wobei das RNA-Ziel eine humane mRNA ist, wie ein mRNA, ausgewählt aus der Gruppe, bestehend aus ApoB-100, CRP, PCSK9, PTP-1 B, GCGR, GCCR, SGLT2, Clusterin, Survivin, eiF-4E, Hsp27, ICAM-1, VLA-4, IL-4Ralpha, C-ref-Kinase, SOD1 und GHr.

8. Antidotverbindung zur Verwendung als Arzneimittel nach einem der Ansprüche 1 - 7, wobei das erste Oligomer gegen PCSK9 oder ApoB gerichtet ist und das erste Oligomer eine Krankheit behandelt, ausgewählt aus der Gruppe, bestehend aus Arteriosklerose, Hypercholesterolämie und Hyperlipidämie.

9. Antidotverbindung zur Verwendung als Arzneimittel nach einem der Ansprüche 1 - 7, wobei das erste Oligomer gegen Hif1 alpha, Survivin, Bcl2, Mcl1, Her3, einen androgenen Rezeptor, Beta-Katenin, Pl3-Kinase oder FABP4 gerichtet ist und das erste Oligomer Krebs behandelt.

10. Antidotverbindung zur Verwendung als Arzneimittel nach einem der Ansprüche 1 - 5, wobei das RNA-Ziel eine humane mikroRNA ist.

11. Antidotverbindung zur Verwendung als Arzneimittel nach einem der Ansprüche 1 - 5 oder Anspruch 10, wobei das erste Oligomer gegen miR-122 gerichtet ist und wobei das erste Oligomer eine Hepatitis-C-Infektion behandelt.

12. Antidotverbindung zur Verwendung als Arzneimittel nach einem der Ansprüche 1 - 11, wobei das zweite Oligomer ein Mixmer ist, das sowohl natürlich vorkommende Nukleoside als auch LNA-Nukleotidanaloga umfasst.

13. Antidotverbindung zur Verwendung als Arzneimittel nach einem der Ansprüche 1 - 11, wobei das zweite Oligomer ein Totalmer ist, wobei alle Nukleotide Nukleotidanaloga sind, ausgewählt aus LNA, 2'-O-Alkyl-RNA, 2'-OMe-RNA, 2'-Amino-DNA, 2'-Fluor-DNA, 2'-MOE-RNA.

14. Antidotverbindung zur Verwendung als Arzneimittel nach einem der Ansprüche 1 - 13, wobei die im zweiten Oligomer vorhandenen Internukleosidverbindungen Phosphorthioatverbindungen sind.

15. Kit zur therapeutischen Verwendung, umfassend ein erstes Oligomer in einer ersten Kammer und ein zweites Oligomer in einer zweiten Kammer, wobei die Oligomere nach einem der Ansprüche 1 - 14 definiert sind.

## Revendications

1. Composé antidote, le deuxième oligomère, comprenant un oligomère simple brin d'une longueur de 6 à 30 nucléotides contigus avec au moins un analogue de nucléotide, pour utilisation comme médicament pour désactiver un premier oligomère thérapeutique *in vivo* chez un sujet, dans lequel
a) le premier oligomère est complémentaire ou essentiellement complémentaire d'un acide nucléique cible naturellement présent chez le sujet et le premier oligomère comprend un ou plusieurs analogues de nucléotides choisis parmi : acide nucléique verrouillé (LNA), 2'-O-alkyl-ARN, 2'-OMe-ARN, 2'-amino-ADN, 2'-fluoro-ADN, 2'-MOE-ARN ; et
b) la séquence de nucléotides contigus dudit deuxième oligomère est i) totalement complémentaire d'une séquence de nucléotides contigus présente dans le premier oligomère sur la longueur du plus court des deux oligomères, ou ii) comprend au plus un seul mésappariement avec le complément d'une séquence de nucléotides contigus présente dans le premier oligomère sur la longueur du plus court des deux oligomères et ledit deuxième oligomère comprend un ou plusieurs analogues de nucléotides choisis parmi : LNA, 2'-O-alkyl-ARN, 2'-OMe-ARN, 2'-amino-ADN, 2'-fluoro-ADN, 2'-MOE-ARN et est conçu de manière à ce que le duplex entre le premier oligomère et le deuxième oligomère complémentaire aboutisse à la formation d'au moins une paire de bases d'analogues de nucléotides ; et
c) le deuxième oligomère ne comprend pas de séquence contiguë de plus de trois unités d'ADN.

2. Le composé antidote pour utilisation comme médicament selon la revendication 1, dans lequel les nucléotides 5' et 3' du deuxième oligomère sont des nucléotides de LNA.

3. Le composé antidote pour utilisation comme médicament selon la revendication 1 ou 2, dans lequel la paire d'analogues de nucléotides formée entre le premier et le deuxième oligomère est choisie parmi LNA/LNA, LNA/2'MOE, LNA/2'fluoro, 2'MOE/LNA ou 2'fluoro/LNA (premier oligomère/deuxième oligomère).

4. Le composé antidote pour utilisation comme médicament selon l'une quelconque des revendications 1 à 3, dans lequel le premier oligomère possède une séquence de nucléotides contigus qui i) est totalement complémentaire d'une sous-séquence de nucléotides contigus présente dans un ARN cible de mammifère, ou ii) comprend au plus un seul mésappariement avec le complément d'une sous-séquence de nucléotides contigus présente dans ledit ARN cible.

5. Le composé antidote pour utilisation comme médicament selon l'une quelconque des revendications 1 à 4, dans lequel ladite séquence de nucléotides contigus dudit premier oligomère possède une longueur comprise entre 8 et 30 nucléotides.

6. Le composé antidote pour utilisation comme médicament selon l'une quelconque des revendications 1 à 5, dans lequel ledit premier oligomère est un oligonucléotide gapmère.

7. Le composé antidote pour utilisation comme médicament selon l'une quelconque des revendications 1 à 6, dans lequel ledit ARN cible est un ARNm humain, tel qu'un ARNm choisi dans le groupe consistant en : ApoB-100, CRP, PCSK9, PTP-1 B, GCGR, GCCR, SGLT2, clusterine, survivine, eiF-4E, Hsp27, ICAM-1, VLA-4, IL-4Ralpha, C-ref kinase, SOD1 et GHr.

8. Le composé antidote pour utilisation comme médicament selon l'une quelconque des revendications 1 à 7, dans lequel le premier oligomère cible PCSK9 ou ApoB et le premier oligomère traite une maladie choisie dans le groupe consistant en athérosclérose, hypercholestérolémie et hyperlipidémie.

9. Le composé antidote pour utilisation comme médicament selon l'une quelconque des revendications 1 à 7, dans lequel le premier oligomère cible Hif1 alpha, la survivine, Bcl2, Mcl1, Her3, un récepteur des androgènes, la bêta-caténine, la Pl3 kinase ou FABP4, et le premier oligomère traite le cancer.

10. Le composé antidote pour utilisation comme médicament selon l'une quelconque des revendications 1 à 5, dans lequel ledit ARN cible est un microARN humain.

11. Le composé antidote pour utilisation comme médicament selon l'une quelconque des revendications 1 à 5 ou la revendication 10, dans lequel le premier oligomère cible miR-122, et dans lequel le premier oligomère traite une infection par l'hépatite C.

12. Le composé antidote pour utilisation comme médicament selon l'une quelconque des revendications 1 à 11, dans lequel le deuxième oligomère est un mixmère qui comprend des nucléosides naturels et des analogues de nucléotides de LNA.

13. Le composé antidote pour utilisation comme médicament selon l'une quelconque des revendications 1 à 11, dans lequel le deuxième oligomère est un totalmère dont tous les nucléotides sont des analogues de nucléotides choisis parmi : LNA, 2'-O-alkyl-ARN, 2'-OMe-ARN, 2'-amino-ADN, 2'-fluoro-ADN, 2'-MOE-ARN.

14. Le composé antidote pour utilisation comme médicament selon l'une quelconque des revendications 1 à 13, dans lequel les liaisons internucléosidiques présentes dans le deuxième oligomère sont des liaisons phosphorothioate.

15. Kit à usage thérapeutique, comprenant un premier oligomère dans un premier compartiment et un deuxième oligomère dans un deuxième compartiment, dans lequel les oligomères sont définis selon l'une quelconque des revendications 1 à 14.
